Europäisches Patentamt

European Patent Office

Office européen des brevets

(11) Publication number: **0 520 029 B1**

# EUROPEAN PATENT SPECIFICATION

(45) Date of publication of patent specification: **24.05.95**

(51) Int. Cl.⁶: **C12N  15/55**, C12N 9/16, A61K 48/00, C12N 15/85

(21) Application number: **91906924.5**

(22) Date of filing: **14.03.91**

(86) International application number: **PCT/US91/01748**

(87) International publication number: **WO 91/13989 (19.09.91 91/22)**

Divisional application 94109172.0 filed on 14/03/91.

(54) **METHODS AND COMPOSITIONS FOR THE TREATMENT OF MALIGNANCIES IN WHICH A PROTEIN KINASE IS ASSOCIATED.**

(30) Priority: **14.03.90 US 494036**

(43) Date of publication of application: **30.12.92 Bulletin  92/53**

(45) Publication of the grant of the patent: **24.05.95 Bulletin  95/21**

(84) Designated Contracting States: **AT BE CH DE DK ES FR GB GR IT LI LU NL SE**

(56) References cited:

**Proc. Natl. Acad. Sci. USA, vol. 87, September 1990, (Washington, US), D.E. Cool et al. pp.7280-7284**

**Proc. Natl. Acad. Sci. USA, vol. 86, July 1989, (Washington, US), D.E. Cool et al. pp. 5257-5261**

(73) Proprietor: **WASHINGTON RESEARCH FOUNDATION**
**4225 Roosevelt Way N.E.**
**Suite 303**
**Seattle, WA 98105 (US)**

(72) Inventor: **FISCHER, Edmond, H.**
**5540 N.E. Windermere Road**
**Seattle, WA 98105 (US)**
Inventor: **KREBS, Edwin, G.**
**1153 21st Avenue E.**
**Seattle, WA 98112 (US)**
Inventor: **TONKS, Nicholas, K.**
**159 Harbor Road, Cold Spring Harbor**
**New York 11724 (US)**
Inventor: **COOL, Deborah, E.**
**5400 42nd Avenue W.**
**Seattle, WA 98199 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Biochemistry, vol. 27, no. 24, 29 November 1988, American Chemical Society, (Easton, PA, US), N. K. Tonks et al. pp. 8695-8701

Proc. Natl. Acad. Sci. USA, vol. 83, May 1986, Biochemistry, (Washington, US), S.F. Yu et al. pp. 3194-3198

(74) Representative: **Brown, John David et al**
**FORRESTER & BOEHMERT**
**Franz-Joseph-Strasse 38**
**D-80801 München (DE)**

## Description

Technical Field

The present invention is generally directed toward compositions for use within methods for the treatment of malignancies in which a protein tyrosine kinase is associated. This invention is more particularly related to cancer therapies using DNA sequences encoding protein tyrosine phosphatase or mutant forms of the enzyme.

Background of the Invention

Despite enormous investments of financial and human resources, cancer remains one of the major causes of death. A common characteristic of malignancies is uncontrolled cell growth. Cancer cells appear to have undergone a process of transformation from the normal phenotype to a malignant phenotype capable of autonomous growth. Mutation of somatic cell genes is considered to be a common primary event that results in the transformation of normal cells to malignant cells. The malignant phenotypic characteristics encoded by the mutated genes are passed on during cell division to the progeny of the transformed cells. Various genes involved with transformation have been designated as oncogenes. Oncogenes were originally identified as components of the genetic material of oncogenic viruses. The homologous genes on human chromosomes are commonly termed oncogenes or proto-oncogenes.

Numerous oncogenic viruses appear to operate by encoding a protein tyrosine kinase. This enzyme catalyzes the phosphorylation of tyrosyl residues in proteins. Changes in the state of phosphorylation of tyrosyl residues in proteins have been suggested to be involved in oncogenic transformation. The identity of the protein substrates and the mechanism by which their phosphorylation mediates phenotypic responses remain to be elucidated.

Approaches to the development of cancer therapies have, in general, centered on the use of characteristic differences between normal and malignant cells. More specifically, comparisons of normal and cancer cells have focused on transformation-dependent changes in the molecules residing at the surface of cell membranes. These molecules include glycolipids, proteins and glycoproteins. Certain molecules have been found to disappear or at least decrease greatly upon oncogenic transformation, while other molecules increase. Despite advances in this area, treatment of cancer cells by targeting tumor-associated cell surface molecules has met with limited success.

Due to the difficulties in the current approaches to cancer therapy, there is a need in the art for improved methods and compositions. The present invention fills this need, and further provides other related advantages.

Summary of the Invention

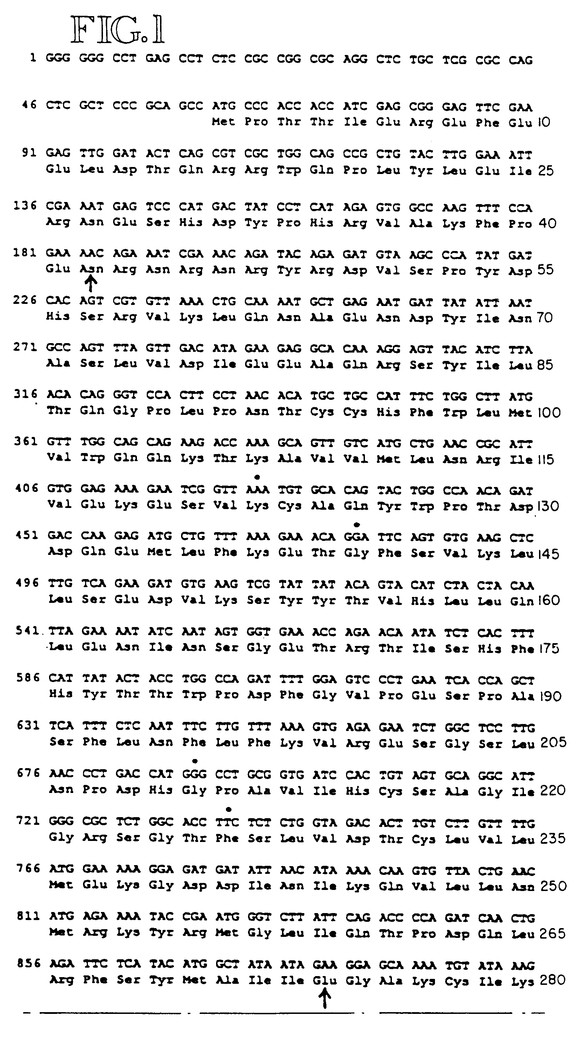

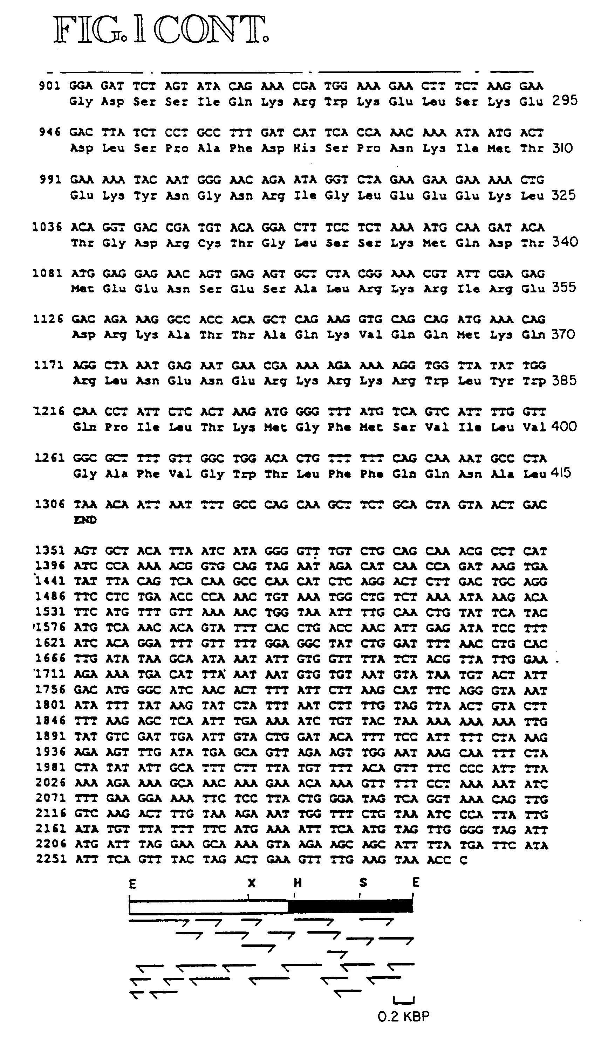

Briefly stated, the present invention provides compositions whose uses include within methods for treating a malignancy in a warm-blooded animal, wherein a protein tyrosine kinase is associated with the malignancy. In one aspect, the composition comprises a gene transfer vehicle capable of infecting malignant cells, wherein the gene transfer vehicle carries a DNA construct comprising a DNA molecule encoding a carboxyl-terminus-truncated non-receptor-linked protein tyrosine phosphatase. The DNA molecule encodes a non-receptor-bed protein tyrosine phosphatase having a truncated carboxyl terminus. In one embodiment, the DNA molecule encodes a carboxyl-terminus-truncated non-receptor-linked protein tyrosine phosphatase, wherein the protein tyrosine phosphatase comprises an amino acid sequence of between 297 to 321 amino acid residues, and wherein the amino acid sequence contains a portion of similar length to and having at least approximately 80% sequence similarity with the amino acid sequence of Figure 1 from asparagine, amino acid 42, to glutamic acid, amino acid 274. There is disclosed a composition comprises a gene transfer vehicle capable of infecting malignant cells, wherein the gene transfer vehicle carries a DNA construct comprising a DNA molecule encoding a receptor-linked protein tyrosine phosphatase. In one example of this the DNA molecule encodes a receptor-bed protein tyrosine phosphatase having a truncated carboxyl terminus. Preferred gene transfer vehicles for the invention and the above disclosure include a recombinant retrovirus or a recombinant vaccinia virus

In another aspect, the present invention provides a variety of isolated DNA molecules encoding non-receptor-linked protein tyrosine phosphatases having truncated carboxyl termini. In one embodiment, the DNA molecule encodes a carboxyl-terminus-truncated non-receptor-linked protein tyrosine phosphatase, wherein the protein tyrosine phosphatase consists essentially of an amino acid sequence of between 297 to 320 amino acid residues, and wherein the amino acid sequence contains a portion of similar length to and having at least approximately 80% sequence similarity with the amino acid sequence of Figure 1 from asparagine, amino acid 42, to glutamic acid, amino acid 274. In another embodiment, the DNA molecule encodes a carboxyl-terminus-truncated non-receptor-linked protein tyrosine phosphatase, wherein the protein tyrosine phosphatase consists essentially of the amino sequence of Figure 1 from methionine, amino acid 1, to an amino acid positioned between leucine, amino add 297, and arginine, amino acid 317. In a related embodiment, the DNA molecule encodes a carboxyl-terminus-truncated non-receptor-linked protein tyrosine phosphatase, wherein the protein tyrosine phosphatase consists essentially of the amino add sequence of Figure 1 from methionine, amino acid 1, to arginine, amino add 317. In another embodiment, the DNA molecule encodes a carboxyl-terminus-truncated non-receptor-linked protein tyrosine phosphatase, wherein the protein tyrosine phosphatase consists essentially of the amino acid sequence of Figure 1 from methionine, amino acid 1, to an amino acid positioned between glutamic acid, amino add 376, and serine, amino acid 396.

These and other aspects of the present invention will become evident upon reference to the following detailed description and attached drawings.

## Brief Description of the Drawings

Figure 1 depicts the sequencing strategy, nucleotide sequence, and deduced amino add sequence of human T cell cDNA encoding protein tyrosine phosphatase (PTPase). The predicted amino add sequence of the open reading frame is shown below the nucleotide sequence. The oligonucleotide sequences used for screening the library are indicated by dots [e.g., between nucleotides 425 and 479 (probe 1), and 689 and 737 (probe 2)]. The TAA stop codon is located at nucleotide 1306 followed by a 3' untranslated end containing two possible polyadenylylation sites AATAAA at 1521 and 1677. The vertical arrows demarcate a 236-residue core segment. The schematic diagram below the nucleotide sequence depicts the sequencing strategy used. Open bar, open reading frame; solid bar, 3' untranslated end. Horizontal arrows indicate the length of sequence obtained from different sequencing oligonucleotide primers. E, EcoRI; H, HindIII, S, Sst I; X, Xba I. The scale at the bottom represents 200 nucleotides (in kbp).

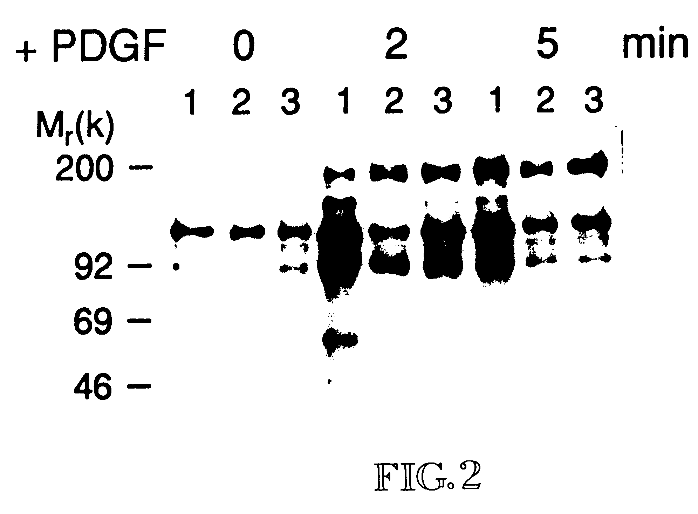

Figure 2 pictorially depicts a Western blot of phosphotyrosine-immunopredpitated proteins following PDGF stimulation Phosphotyrosine-containing proteins and PDGF-stimulated cells were immunoprecipitated with an anti-phosphotyrosine antibody. A SDS/7.5% Laemmli gel was used to separate the precipitated proteins followed by Western blot analysis with an anti-phosphotyrosine antibody as a probe in the blot. Detection of binding was with $^{125}$I-labeled protein A followed by autoradiography at room temperature for 5 days. Lanes: 1, control cells; 2, cells expressing the full-length 48-kDa T-cell PTPase; 3, cells expressing the truncated form. Times of PDGF stimulation following 48 hr of serum-deprivation are indicated (0, 2, or 5 min). Standard molecular mass markers In kDa (200, myosin; 92, phosphorylase B; 69, bovine serum albumin; and 46, ovalbumin) are also shown.

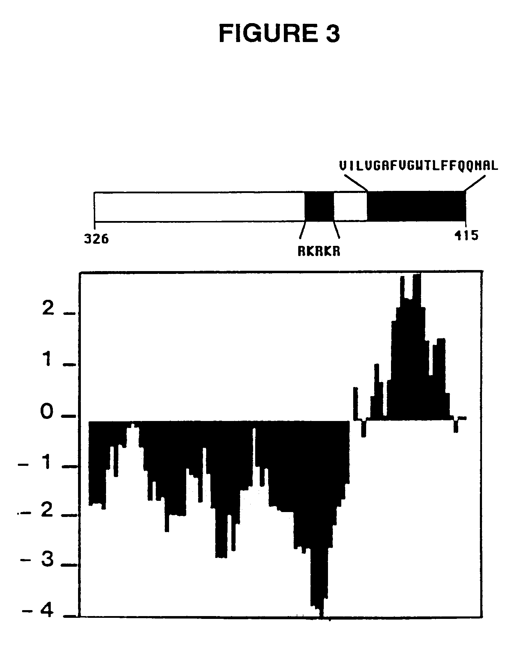

Figure 3 graphically illustrates a hydrophobicity plot of the carboxyl-terminal region in the T-cell PTPbase. The hydrophobicity calculations of amino acid residues 326-415 in the carboxyl-terminal segment of the T-cell PTPbase were determined as described by Kyte and Doolittle (J. Mol. Biol. 157: 105-132, 1982). A computer-generated plot was obtained by using a default window of 7. The numbers above and below the 0 line represent hydrophobic and hydrophilic amino acids, respectively; the units in they axis are defined as in Kyte and Doolittle. The bar above the plot depicts a putative Arg-Lys-Arg-Lys-Arg nuclear recognition signal (Van Etten et al., Cell 58:669-678, 1989) between residues 377 and 381 (solid) and a hydrophobic terminal region (396-415) (solid; 19 residues shown in single-letter code) representing the carboxyl-terminus of the molecule.

## Detailed Description of the Invention

As noted above, the present invention is directed towards compositions whose uses include within methods for treating a malignancy in which a protein tyrosine kinase is associated. The disclosure of the present invention shows the reversion of malignant transformation by transfection with human DNA encoding a carboxyl-terminus-truncated non-receptor-linked protein tyrosine phosphatase.

Within one aspect of the present invention, a malignancy in which a protein tyrosine kinase is associated may be treated with a gene transfer vehicle capable of infecting malignant cells, wherein the gene transfer vehicle carries a DNA construct comprising a DNA sequence encoding a carboxyl-terminus-truncated non-receptor-linked protein tyrosine phosphatase or mutant forms of the enzyme. This therapy is applicable to a wide variety of malignancies found in warm-blooded animals such as humans. Representative malignancies include breast cancer and leukemia Within the present invention it is not necessary for all malignancies to involve tyrosine phosphorylation of the same type of protein substrate, provided that dephosphorylation of tyrosyl residues by a protein tyrosine phosphatase (whose DNA sequence has been incorporated into malignant cells) results in the reversion of the malignant phenotype.

A variety of DNA sequences encoding a protein tyrosine phosphatase ("PTPase") are suitable within the present invention The DNA sequence encodes a carboxyl-terminus-truncated non-receptor-linked PTPase. Non-receptor-linked PTPases typically have a molecular weight less than approximately 50,000. Receptor-linked PTPbases typically have a higher molecular weight, e.g., 150,000-250,000, and display a wide variety of external domains.

A representative example of wild type, non-receptor-linked PTPase DNA is the sequence shown in Figure 1 for human T cell PTPase. Other examples include a DNA sequence encoding a PTPase of human placenta. A representative example of wild type, receptor-linked PTPase DNA is the DNA sequence encoding CD45 in human leukocytes.

The DNA sequence used within the present invention encodes a PTPase having a truncated carboxyl terminus, i.e., missing amino acid residues normally found at or near the carboxyl terminus in the sequence of a wild type PTPase. The disclosure of the present invention provides a representative example of a DNA sequence encoding a truncated PTPase. This DNA was prepared by altering the nucleotide sequence of the cDNA encoding a T cell PTPase (Figure 1) using standard molecular biology techniques. Briefly, a deletion of a few nucleotides by site directed mutagenesis was introduced into wild type cDNA. This sequence alteration resulted in the placement of a premature translation stop signal in the open reading frame of the cDNA. The modified cDNA encodes a PTPase (molecular mass of about 37,000) with a truncated carboxyl terminus relative to the 48,000 molecular mass protein normally produced in T cells. This truncated PTPase has the amino acid sequence of Figure 1 from methionine, amino acid 1, to arginine, amino acid 317. The enzymatic activity exhibited by transfected cells expressing this truncated PTPase is higher than the activity in control cells.

It will be evident to those skilled in the art that a variety of DNA sequences encoding PTPases having truncated carboxyl termini may be employed within the present invention. In general, any number of amino acid residues may be deleted from the carboxyl terminus, provided that the resultant protein does not exhibit a significant reduction in PTPase activity. The enzymatic activity of truncated PTPases may be measured, for example, using an assay based on the measurement of a reporter group released from a labeled substrate. A suitable assay includes the one described below for the measurement of $^{32}$P released from reduced, carboxyamidomethylated maleylated-lysozyme. As denoted in Figure 1, there is a 232-amino acid residue core identified by T cell PTPase amino acid 42 (asparagine) to amino acid 274 (glutamic acid). T cell PTPase and placenta PTPase 1B, for example, possess within this core about 65% sequence identity and approximately 80% sequence similarity (after optimizing the alignment between the two sequences). Truncated forms of PTPase may be prepared by the deletion of amino acids starting at the carboxyl terminus and moving back towards the 236-residue core. Suitable DNA sequences include those encoding a PTPase comprising an amino acid sequence of between (including) 297 to 320 amino acid residues where the sequence contains a portion of similar length to, and having at least approximately 80% sequence similarity with, this 232-residue core. This portion of a sequence need not be of identical length and it may be necessary to shift the sequence and/or insert gaps (in it or the sequence of Figure 1) in order to optimize the alignment between the two sequences. Sequence similarity is based upon sequence identity plus conservative substitutions of amino acids. Conservative substitutions include interchanges of valine and isoleucine, leucine and isoleucine, aspartic acid and glutamic acid, and others of a similar nature. A preferred DNA sequence encodes a truncated PTPase comprising the amino acid sequence of Figure 1 from methionine, amino acid 1, to an amino acid positioned between (including) leucine, amino acid 297, and arginine, amino acid 317.

Following isolation or preparation of a suitable DNA sequence encoding a truncated PTPase, the sequence is inserted into a gene transfer vehicle. Suitable gene transfer vehicles include a recombinant retrovirus or a recombinant vaccinia virus.

Retroviruses are RNA viruses that can replicate through a DNA intermediate through the action of an RNA dependent DNA polymerase. Once in DNA form, they may be stably integrated into the host cell genome and passed down from a parent cell to its progeny. Thus, retroviruses are suitable as transfer vehicles for foreign DNA into a host cell (see Shimotohno and Temin, "Formation of Infectious Progeny Virus Alter Ingestion of *Herpes Simplex* Thymidine Kinase Gene into DNA of an Asian Retrovirus," Cell 26:67-77, 1981; see also, Shimotohno and Temin, "Loss of Intervening Sequences in Genomic Mouse Alpha-Globin DNA Inserted in an Infections Retrovirus Vector," Nature 299:265-268, 1982; see also; Miller and Rosman, "Improved Retroviral Vectors for Gene Transfer and Expression," BioTechniques 7:98-990, 1989).

Where the retrovirus transfer vehicle is replication competent, the resultant active virus may not be appropriate for therapeutic purposes because it is also replication competent, and represents a health hazard. However, various methods have been utilized to design a retrovirus transfer vehicle which can produce a retrovirus containing foreign DNA, yet which is nevertheless replication incompetent (see U.S. Patent Nos. 4,650,754 (Temin et al.), 4,861,719 (Miller), 4,405,712 (Van de Woude et al.), and 4,885,238 (Reddel et al.), which are incorporated herein by reference; see also Jacob et al., European Patent Application Publ. No. 0178220, European Patent Application Publ. No. 0243204, PCT International Publ. No. WO 85/05629, PCT International Publ. No. WO 87/03451, PCT International Publ. No. 89/07150, and PCT International Publ. No. WO 89/09271). Various retroviruses may be used within the present invention including the Murine Sarcoma Virus (MSV) and the Moloney Murine Leukemia Virus (MoMLV). Furthermore, it will be evident to one of ordinary skill in the art that envelope sequences may be derived from amphotropic viruses such as virus 4070A.

Briefly, within one embodiment of the present invention, a mutant of the Moloney murine leukemia virus is constructed which contains a deletion of about 350 nucleotides in the *env* region of the retrovirus (see Shank and Linial, J. Virol. 36(2):450-456, 1980). This produces a virus (gag$^+$,pol$^+$,env$^-$) deficient in encapsidation or packaging of viral RNA, which may, if cotransfected with an encapsidation positive retroviral vector (gag$^-$,pol$^-$,env$^+$) containing a desired foreign gene, result in the production of retrovirus which is replication incompetent, yet which nevertheless produces the foreign gene (see Temin et al. U.S. Patent No. 4,650,764).

There remains, however, the possibility of recombination between the gag$^-$,pol$^-$,env$^+$ vector and the gag$^+$,pol$^+$,env$^-$ vector. Such recombination might produce an infectious retrovirus (gag$^+$,pol$^+$,env$^+$). Therefore, it is particularly preferred to design recombinant retrovirus vectors which are safe transfer vehicles for foreign genes. Briefly, the retroviral genome, or provirus, contains two Long Terminal Repeats (LTR) which encode sequences for initiation and termination of retroviral transcription, including promoters, translation ad termination signals, as well as enhancers. The LTR has 3 regions: U3, R, and U5. Within a preferred embodiment the U3 region (containing promoter and enhancer segments) of the right side LTR is deleted resulting in a retroviral vector which is self-inactivating (see Yu et al., "Self-Inactivating Retroviral Vectors Designed for Transfer of Whole Genes Into Mammalian Cells," Proc. Natl. Acad. Sci. USA 83:3194-3198, 1986; Markowitz et al., "A Safe Packaging tine for Gene Transfer: Separating Viral Genes on Two Different Plasmids," J. Virol. (62):1120-1124, 1988). This vector (containing a promoter and the foreign gene, i.e., U3, R, U5----promoter, gene, ----U3(del), R, U5) is used to transfect helper cells. Viral transcription begins on the left side LTR beginning at R, and transcribes a sequence which does not contain either the left side or right side U3 region (i.e., R, U5, ---- promoter, gene ---- U3(del), R). Thus, the retroviral vector is self-inactivating yet, nevertheless, because it contains an internal promoter allows transcription of the foreign DNA.

Vaccinia viruses are eukaryotic DNA viruses that reproduce entirely within the cytoplasm of a host cell. The virus has been used for about 200 years in vaccines for inoculation against smallpox. The virus is considered non-oncogenic and relatively benign. The naturally occurring vaccinia genome can be modified to produce recombinant vaccinia by rearrangement of the natural genome, by the removal of DNA from the genome, and/or by the introduction of foreign DNA into the genome (e.g., U.S. Patent Nos. 4,769,330 and 4,886,782, which are incorporated herein by reference). Thus, recombinant vaccinia virus represents a relatively innocuous eukaryotic cloning vector useful within the present invention for the introduction of a DNA sequence encoding a PTPase into a eukaryote.

The following examples are offered by way of illustration and not by way of limitation.

**EXAMPLES**

EXAMPLE 1

CONSTRUCTION OF A HUMAN T CELL PROTEIN-TYROSINE-PHOSPHATASE

All restriction and modifying enzymes and *in vitro* transcription and translation systems were purchased from Stratagene, La Jolla, California. Oligonucleotides were synthesized with an Applied Biosystems 380A DNA Synthesizer (Applied Biosystems, Foster City, Calif.). Radionucleotides were obtained from New England Nuclear (Boston, Mass.). Sequenase was obtained from United States Biochemical (Cleveland, Ohio). Solutions such as Denhardt's solution were prepared as described by Maniatis et al., Molecular Cloning: A Laboratory Manual, Cold Spring Harbor Lab., Cold Spring, N.Y., 1982.

## A. Screening of a cDNA Library with Synthetic Oligonucleotides

A set of complementary overlapping oligonucleotides were synthesized for each of the protein segments Lys-120-Asn-139 and Gly-209-Phe-225 from the human placenta PTPase 1B according to the method of L. Charbonneau et al., Proc. Natl. Acad. Sci. USA 86:5252-56, 1989. Prediction of the DNA sequence was based on the optimal codon choice for human amino acid sequence data (see R. Lathe, J. Mol. Biol 183:1-12, 1985). Oligonucleotides in set 1 (5'-AAGTGTGCACAGTACTGGCCGCAGAAGGAA-3' and 5'-GTTGGTATCCTCAAAGATCATCTCCCTTCTCTTCCTTC TGC-3') or in set 2 (5'-GGTCC TGTGGTGGTGCACTGCAGTGCTGGT-3' and 5'-AAGGTTCCAGTG CGCCAATACCAGCACTG-3') were annealed and labeled using $^{32}$PdATP and $^{32}$PdCTP and the Klenow fragment of DNA polymerase I, producing radioactive double-stranded DNA with a specific activity of $4 \times 10^7$ cpm/pmol bp.

A λgt10 recombinant cDNA phage library containing 500,000 phage was prepared from human peripheral T cell poly(A)$^+$ mRNA according to the method of Littman et al., Cell 40:237-46, 1985, and plated at a density of 50,000 phage per plate. Duplicate nitrocellulose filter lifts were taken from each plate and hybridized with the oligonucleotide probes prepared above ($2.5 \times 10^6$ cpm per filter) in 20% formamide/5 x SSC (1 x SSC is 150 mM NaCl/15 mM sodium citrate)/2.5 x Denhardt's solution/1 mM sodium pyrophosphate/50 mM sodium phosphate buffer, pH 6.8, at 37°C for 18 hours. The filters were washed in 2 x SSC/0.2% SDS at 42°C and subjected to autoradiography for 3 days at -70°C with an intensifier screen.

Although many recombinant phage hybridized to each probe, only one overlapping positive clone bound to both oligonucleotides.

## B. DNA Sequence Analysis

Approximately $10^{10}$ E. coli cells were infected with the recombinant phage selected above, and lysed by the alkali method (see Sambrook et al., Molecular Cloning: A laboratory Manual, 2 ed., Cold Spring Harbor laboratory Press, pp. 1.25-1.28, 1989). The amplified phage were purified by CsCl density centrifugation. DNA from the purified phage was extracted and digested with EcoRI restriction endonuclease. The EcoRI fragments were analyzed by agarose gel electrophoresis which resolved two vector DNA fragments (32 and 11 kbp) and a cloned cDNA fragment of 2.3 kilobase pairs (kbp) in length.

One hundred nanograms of the 2.3 kbp EcoRI insert and 100 ng of the Bluescript cloning vector (Strategene, La Jolla, Calif.) were cleaved with EcoRI (a site within the β-galactosidase gene), ligated in a reaction mixture containing 50 mM Tris (pH 7.4), 10 mM MgC$_2$, 10 mM dithiotheritol and 2 mM ATP, and transformed into an Escherichia coli host (XL-Blue, Stratagene). The transformed cells were plated on agar plates containing 100 μg/ml ampicillin and 50 μg/ml X-gal (Bethesda Research Lab). White colonies (containing cDNA inserts) were selected and a miniprep of plasmid DNA was prepared and run on an agarose gel to ensure presence of an insert. A cell was selected which contained the insert and grown in LB (Luria-Bertani) medium (see Sambrook et al. supra, p.A.1) for 12 hours. Cells were lysed by the alkali method, and the closed circular plasmid DNA extracted and purified by CsCl centrifugation.

The purified DNA was sequenced using the strategy illustrated in Figure 1 according to the chain-termination method of Sanger et al. (Proc. Natl. Acad. Sci. USA 74:5463-6467, 1977). Briefly, sequencing reactions contain a mixture of deoxy (including $^{32}$PdATP) and dideoxy nucleotides. The DNA to be sequenced is denatured by boiling and annealed with 18-mer single stranded primers which recognize a precise sequence in the cDNA or vector sequence adjacent to the cloning site. The DNA is copied from the priming site utilizing the Sequenase enzyme provided in a sequencing kit (United States Biochemicals Co., Cleveland, Ohio). The exact ratios of deoxy and dideoxy nucleotides have been determined by the supplier and were used accordingly.

Sequence analysis (Figure 1) shows that the T cell PTPase cDNA contains an open reading frame of 1305 nucleotides. A consensus sequence CC(AG)CCAUG(G) for eukaryotic initiation sites (described by Kozak, Nucleic Acids Res. 12:857-873, 1984) was found at nucleotides 56-64 encoding a putative initiator methionine. The open reading frame terminates with a TAA stop codon followed by 978 bp of 3' untranslated end. However, neither a polyadenylylation site nor a 3' poly(A)$^+$ tail was observed. There are two possible AATAAA polyadenylylation signals (see Proudfoot and Brownlee, Nature 265:211-214, 1976) at sites 213 and 369 bp past the stop codon (nucleotides 1521-1526 and 1677-1682, respectively).

## C. In Vitro Translation of T-Cell PTPase mRNA

The Bluescript plasmid containing the T cell cDNA was made linear by HindIII restriction endonuclease digestion. mRNA was synthesized in vitro from 1 μg of plasmid DNA using the T7 polymerase promoter,

7

which is positioned 5' to the T cell PTPase cDNA insert in the Bluescript cloning vector. Synthesis of mRNA was carried out in the presence of DNA, nucleotide triphosphates required for RNA, buffer and T7 Polymerase (all reagents were provided by Strategene). Following synthesis for 1 hour at 37°C, the DNA was degraded with DNAse I (Stratagene), and the proteins digested with Proteinase K (Stratagene) for 1 hour at 68°C. RNA was then extracted with phenol:chloroform (1:1) and ethanol precipitated. The mRNA (1 $\mu$g) was added to 20 $\mu$l of a rabbit reticulocyte translation system in the presence of $^{35}$S methionine and protein synthesis was allowed to proceed for 30 minutes. The control reaction mixture contained mRNA produced from the linearized vector. The products were analyzed on a 10% SDS-polyacrylamide gel according to the method of Laemmli (Nature 227:680-685, 1970) and subjected to autoradiography for 18 hours. A protein product with an estimated $M_r$ of 48,000 was produced.

D. Northern Blot Analysis

Total RNA was extracted according to the method of Cathala et al., DNA 2:329-335, 1983, from monkey brain, spleen, and thymus; human RNA was extracted from placenta and T cells. Poly(A)$^+$ mRNA was purified by oligo(dT) column chromatography according to the method of Maniatis et al, *supra*, p. 197. Poly-(A)$^+$ mRNA (10 $\mu$g) from brain, spleen, thymus, and placenta and 20 $\mu$g of the total T cell mRNA were subjected to electrophoresis in a 0.8% formaldehyde agarose gel and transferred to nitrocellulose filter paper by the diffusion method described by Maniatis et al., *supra*, p. 203. The filter containing RNA (Northern blot) was hybridized and denatured. $^{32}$P-labeled cDNA insert purified from the T cell clone was used as a probe. The hybridization conditions were the same as those described for the screening of the library, except that the blot was washed in 0.1 x SSC/0.2% SDS at 50°C. The gel was exposed to film for 3 days at -70°C with an intensifier screen.

Analysis of the blot revealed multiple bands of hybridization. The most abundant transcript ($\approx$2.3 kb) was found in all the above tissues, although the level of expression in brain was quite low. Comparison of the thymus poly(A)$^+$ mRNA with the T cell total mRNA showed at least a 20-fold enrichment of the transcript. The predominant message, whose precise length cannot be determined in the agarose gel, appears to represent the T cell PTPase cDNA since the expected length of this transcript is at least 2.5 kb including a 200-base poly(A)$^+$ tail (see Perry, Annu. Rev. Biochem. 45:605-629, 1976).

E. Southern Blot Analysis

Human genomic DNA was cleaved with the restriction endonucleases BamHI, EcoRI, and HindIII. The fragments were separated in a 0.8% agarose gel and transferred to nitrocellulose filter paper by diffusion (see Maniatis, *supra*). The filter containing DNA (Southern blot) was hybridized to the denatured $^{32}$P-labeled insert of the T cell cDNA and washed as described for the Northern blot analysis and subjected to autoradiography for 3 days at -70°C with an intensifier screen. Autoradiography of the blot revealed several bands of hybridization, indicating that either the gene is very large (>70 kbp with many introns) or that there are multiple genes in this family. It was then reprobed with the labeled cDNA using the same hybridization conditions as above, but washed under less stringent conditions, such as 2 x SSC/0.2% SDS and 45°C. No new bands of hybridization were detected.

F. Insertion of the Human T Cell PTPase Clone Into a Plasmid

An EcoRI-HindIII fragment (1.328 kbp) was isolated from the human T cell cDNA PTPase clone obtained above. This fragment represents the entire coding region of the PTPase cDNA; including 60 b.p. from the 5' untranslated region and 22 b.p. from the 3' untranslated region. The single stranded ends produced by the restriction enzymes were removed with nuclease S1 digestion.

Many plasmid vectors are known to one of ordinary skill in the art which are suitable for expressing DNA inserts, including pMFG (Pharmacia LKB Biotechnology, Inc., Piscataway, N.J.), and pNut (obtained from Richard Palmiter, University of Washington; see Palmiter et al., Cell 50:435-443, 1987). The pNut expression vector was cleaved with SmaI and run on an agarose gel. A 5.5 kbp fragment was isolated from the agarose gel by electroelution of the DNA into buffer. The DNA was then precipitated with ethanol. The fragment encodes a dihydrofolate reductase cDNA under the regulation of a simian virus 40 (SV40) promoter and a Zn$^{2+}$ metallothioneine I promoter required for in vivo transcription of the newly inserted cDNA The plasmid pNUT.TCPTP was generated by ligating the 1.3 kbp T cell PTPase cDNA fragment with the SmaI pNUT vector fragment.

8

EXAMPLE 2

CONSTRUCTION OF A TRUNCATED FORM OF THE T-CELL PTPASE

A mutation of the PTPase cDNA was performed according to the method of Kunkel et al., "Rapid and Efficient Site-Specific Mutagenesis Without Phenotypic Selection," Methods in Enzymology 154:367-382, 1987. Briefly, the 2.3 kbp cDNA EcoRI insert was ligated into the EcoRI site of an M13 mp18 vector. The ligated DNA was transformed into an E. coli host and M13 phage plaques were produced on an agarose plate. The DNA of these phage plaques is single stranded and can be used for DNA sequencing or for site directed mutagenesis. In particular, the DNA was purified (see Sanger, supra) and hybridized to the oligonucleotide 5'-GGG AAC AGA TAG AAG AAG-3'. The oligonucleotide was synthesized with an Applied Biosystems 380A DNA Synthesizer, and represents nucleotides 1004-1025 with a seven base deletion in the wild type cDNA resulting in the placement of a stop codon (TAG) into the translation open reading frame. The primed, single stranded M13 DNA was used as a template to generate double stranded DNA, one of which had the deletion. The newly synthesized heterodimer double stranded DNA was transformed into E. coli host and plated on agarose plates to allow the growth of M13 recombinant phage. M13 phage carrying the deletion were selected by in situ plaque filter hybridization (Wood et al., Proc. Natl. Acad. Sci. USA 82:1585-1588, 1985) with $^{32}$P-labeled oligonucleotide, followed by washing 6 x SSC, at 48°C. A 1.6 kbp fragment was produced upon ThaI and SspI restriction enzyme digestion of the mutated M13 plasmid DNA. The fragment was isolated and inserted into the SmaI site of the pNUT expression vector. All plasmid constructs were verified by DNA sequence analysis using the chain termination method of Sanger et al., supra.

EXAMPLE 3

PTPASE ASSAYS

Cell extracts or purified enzymes were assayed for tyrosine phosphatase using a method described by Tonks et al., in "Purification of the Major Protein-tyrosine-phosphatases of Human Placenta," J. Biol. Chem. (263):6722-6730, 1988, based on measurement of the release of $^{32}$P from labeled substrate. Since phosphorylation of reduced, carboxyamidomethylated, maleylated (RCM)-lysozyme is not stoichiometric, concentrations are expressed in terms of $^{32}$P phosphotyrosine. Briefly, a mixture of 0.02 ml of PTPase (diluted to less than 1 unit/ml in Buffer A) and 0.02 ml of Buffer A was warmed at 30°C for 5 minutes. Buffer A is composed of 25 mM imidazole HCl (pH 7.2), 1 mg/ml BSA, 0.1% (v/v) $\beta$-mercaptoethanol. The reaction was initiated by addition of 0.02 ml of $^{32}$P-TyrRCM lysozyme (final concentration of 5 $\mu$M) that had also been preincubated to 30°C. The reaction was terminated after approximately 10 minutes by addition of 0.18 ml of 20% (w/v) trichloroacetic acid and 0.02 ml of 25 mg/ml BSA, added as a carrier protein. The suspension was vortexed, allowed to stand on ice for 10 minutes, and centrifuged at 12,500 x g for 3 minutes. A 0.2-ml aliquot of the supernatant was added to 1 ml of Aquasol scintillant and counted in a Beckman LS7000 scintillation counter. Dephosphorylation was linear with respect to time and enzyme concentration; up to 50% of the $^{32}$P was released. Blank incubations were performed in which the PTPase was replaced by Buffer A, and total $^{32}$P was determined by counting 0.02 ml of the substrate. Radioactivity in the blank was routinely less than 2% of total $^{32}$P in the assay. Released $^{32}$P was confirmed to be inorganic phosphate, rather than radioactive peptides, by the molybdate/isobutyl alcohol/benzene extraction procedure of Foulkes et al., FEBS Lett. 130:197-200, 1981. One unit of PTPase activity is defined as that amount which releases 1 nmol of phosphate/min.

For those assays requiring trypsin, 20 $\mu$l of cell extract was diluted 1:2 in buffer and treated with 1 $\mu$g of trypsin for 5 min. at 30°C. Trypsin digestion was stopped by adding 6 $\mu$g of lima bean trypsin inhibitor, followed immediately by 20 $\mu$l of substrate.

EXAMPLE 4

PREPARATION OF ANTI-PEPTIDE SERA

The peptide CNRNRYRDVSPFDHSRIK (identified by reference to the single-letter amino acid code) was synthesized on an Applied Biosystems Peptide Synthesizer (Applied Biosystems, Foster City, Calif.). The sequence was derived from an amino terminal region (residues Asn 43 to Lys 60 of placenta PTPase 1B enzyme (see Charbonneau et al., Proc. Natl. Acad. Sci. USA 86:5252-5256, 1989). The peptide contains an additional cysteinyl residue to facilitate cross-linking to rabbit serum albumin as a carrier protein. Polyclonal antibodies were produced by immunization of a rabbit using conventional techniques followed by collection of its sera. The serum was affinity purified or was loaded onto a cellulose Affi-Gel Blue/DE 52 column (mixed 1:1) and eluted in 20 mM Tris pH 8.0, 20 mM NaCl. Fractions were collected and analyzed for protein. The antibody still recognizes the T cell enzyme even though two amino acid substitutions are found in this domain (tyrosine for Phe-52 and valine for Ile-57). Specificity of the antibody for PTPase was verified by peptide competition experiments.

EXAMPLE 5

EXPRESSION OF WILD-TYPE AND TRUNCATED T-CELL PTPASE

A. BHK Cells

Baby Hamster Kidney (BHK) cells were routinely grown in Dulbecco's modified Eagle's medium containing 10% (vol/vol) heat-inactivated fetal calf serum. The cells were transfected with 10 $\mu$g of the pNut plasmid containing either the T cell PTPase DNA or the truncated T cell PTPase DNA using the calcium phosphate precipitation method (Wigler et al., Cell 16:772-785, 1974) and after 24 hours were switched to selection media containing 250 $\mu$M methotrexate. Stable colonies were isolated about 14 days post transfection.

Confluent stably transfected cells were treated with 80 $\mu$M ZnSO$_4$ for 12 hours in order to induce transcription of the PTPase in RNA through the metallothionine promoter. The plates were washed three times with phosphate-buffered saline (PBS) and scraped in order to remove the cells. The cells were pelleted by low speed centrifugation (500 xg) for 5 min. and the pellet lysed in either a low salt buffer (LSB) (25 mM Imidizole (pH 7.0), 2 mM MgCl$_2$, 1 mM EDTA, 1 mM EGTA, 0.1% $\beta$-mercaptoethanol, 0.002% PMSF, 0.1 mM Benzamidine, 1 $\mu$g/ml leupeptin, 250 mM sucrose), LSB-Triton X-100 buffer, (LSB and 0.5% Triton X-100) or KCl/CHAPS buffer (KCB) (same as LSB without sucrose and including 0.6M KCl, 1.0% CHAPS). The homogenates were dounced for 30 seconds with a Teflon® homogenizer, followed by centrifugation at 5,000 xg for 5 min. to yield a low-speed centrifugation pellet ("5P"). The supernatant was recentrifuged at 100,000 xg at 4°C for 30 minutes to yield a high-speed centrifugation supernate ("100S") and pellet ("100P").

PTPase assays (as described in Example 3) were carried out on the various fractions (Table 1).

TABLE 1

| Fraction | Transfected plasmid | Total Units | | Units/mg | | Fold trypsin stimulation | % total units | |
|---|---|---|---|---|---|---|---|---|
| | | - | + | - | + | | - | + |
| A low-speed pellet (5 P) | Control | 5.2 | 31.0 | 0.7 | 4.4 | 6.0 | 73 | 87 |
| | TC.PTPase | 11.3 | 320.0 | 1.6 | 31.0 | 19.0 | 84 | 97 |
| | TQΔC11.PTPase | 17.0 | 26.0 | 2.5 | 3.7 | 1.5 | 48 | 66 |
| B High-speed pellet (100 P) | Control | 0.4 | 0.5 | 0.6 | 2.6 | 4.5 | 5 | 2 |
| | TC.PTPase | 1.0 | 5.6 | 1.0 | 5.6 | 5.0 | 5 | 2 |
| | TCΔC11.PTPase | 3.4 | 3.9 | 4.3 | 4.9 | 1.0 | 9 | 10 |
| C High-speed supernatant (100 S) | Control | 1.6 | 4.0 | 0.4 | 1.1 | 3.0 | 22 | 11 |
| | TC.PTPase | 2.1 | 5.4 | 0.6 | 1.5 | 2.5 | 11 | 1 |
| | TCΔC11.PTPase | 12.5 | 7.5 | 3.4 | 2.1 | 0.6 | 43 | 24 |

BHK cells were fractionated (5P, 100P, or 100S fractions) by centrifugation as described. The PTPase was determined in cells expressing either the control plasmid (control) or the cDNA of the full-length 48-kDA T-cell PTPase (TC.PTPase) or the truncated form (TCΔC11.PTPase). Total units of activity have been standardized to a constant amount of protein in each fraction. The signs "-"or " + " indicate assays without or with 1 $\mu$g of trypsin in the assay, respectively. "% total units" represents the percentage of total cellular activity found in each fraction.

Essentially all of the endogenous and expressed PTPase activities (of BHK cells expressing a full-length 48-kDa human T cell PTPase) sedimented with the 5P pellet from which they could be released by 0.5% Triton X-100/0.6 m KCl. Triton alone was only partially effective, and salts alone at high concentration were totally ineffective. The low levels of activity ad protein found in the 100P pellet was not considered further. Although the level of enzyme in the transfected cells was found at first to be no greater than in the controls, it could be increased considerably upon limited trypsinization (6- and 20-fold in the 5P fraction for the control and transfected cells, respectively). Under these conditions, the total activity in the transfected cells was 10 times greater than that in the controls.

A 0.5% Triton X-100 extract was subjected to Superose 12 fast protein liquid chromatography (FPLC) gel filtration. In both control and transfected cells, the PTPase activity emerged in a high molecular mass (>650 kDa) fraction. Western blot analysis of this material following $CCl_3$ COOH precipitation ad SDS/PAGE revealed the presence of a 48-kDa immunoreactive protein. Similar results were obtained when the cells were extracted in 1% 3-[(3-cholamidopropyl)dimethylammonio]-1-propanesulfonate (CHAPS)/0.6 M KCl and then freed of detergent by dialysis against a low-salt buffer prior to gel filtration, suggesting that the formation of the high molecular mass complex was not due to the presence of detergents.

When the Triton-soluble extracts were treated with trypsin prior to gel filtration, the PTPase was eluted with an apparent molecular mass of approximately 35 kDa. Western blot analysis also revealed a bad at approximately 33 kDa, suggesting that cleavage had occurred at the carboxyl terminus, since the antibody used recognized a sequence near the amino terminus of the enzyme. Pretrypsinization of the extract from the control cells also resulted in a new peak of activity that was eluted in fractions containing low molecular mass proteins. The above data indicate that removal of a carboxyl-terminal segment from the enzyme by trypsin treatment results in the formation of a water-soluble, low molecular mass, constitutively active enzyme.

When extracts of BHK cells expressing a truncated PTPase in which an 11-kDa segment was deleted from the carboxyl end (TCΔC11.PTPase-transfected cells) were fractionated as described above ad assayed without prior trypsin treatment, only about 50% of the PTPase activity sedimented with the 5P fraction, while the remainder was in the 100S supernatant (Table 1). However, the enzyme present in the 5P pellet was fully active without trypsin treatment and could be extracted with either 0.5% Triton X-100 or 0.6 M KCl, indicating that it was not as tightly associated with the particulate fraction as was the full-length PTPase. Although the total phosphatase activity in the cells expressing the truncated enzyme was the same as that in the controls, it differed in that it was 8-fold higher in the 100S fraction. When BHK cells expressing the truncated PTPase were extracted with the Triton buffer ad subjected to Superose 12 gel filtration, little of the activity distributed with the high molecular mass complex; indeed, it was detected only in low molecular mass fractions of approximately 35 kDa as confirmed by Western blot analysis. BHK cells transfected with this truncated form of the PTPase exhibited only about 50% of the growth rate of the control cells or cells transfected with the wild-type enzyme.

To determine the state of activity of the enzyme *in vivo*, cells expressing both forms of the T-cell PTPase were stimulated with PDGF, and changes in protein-tyrosine phosphorylation were investigated. The BHK cells were treated with 80 $\mu$M $ZnSO_4$, followed by a 48-hour incubation in medium and in 0.1% heat-inactivated Fetal Calf Serum for 48 hours, which renders the cells quiescent. Serum-deprived cells were treated with 40 ng of platelet-derived growth factor (PDGF) (Amgen Biologicals) per ml at 37°C for various times and then washed immediately with ice-cold PBS. Lysis buffer (1 ml) containing 50 mM Hepes (pH 7.5), 150 mM NaCl, 10% (vol/vol) glycerol, 1% Triton X-100, 1.5 mM $MgCl_2$, 1 mM EGTA, 10 $\mu$g of aprotinin per ml, 2 $\mu$g of leupeptin per ml, 0.002% phenylmethylsulfonyl chloride, 200 $\mu$M sodium orthovanadate, 10 mM sodium pyrophosphate, and 100 mM NaF was added to the plates, which were then incubated on ice for approximately 20 min. The lysates were centrifuged at 10,000 x g for 5 min. at 4°C, and the protein concentrations were determined as described by Bradford (Anal. Biochem. 72:248-254, 1976). Thirty microliters of a suspension of agarose-linked mouse monoclonal anti-phosphotyrosine antibody beads (Oncogene Science, Manhasset, N.Y.) was added to equivalent amounts of lysate protein in each immunoprecipitation, and the mixture was rotated overnight at 4°C. The beads were collected by centrifugation, washed twice in 20 mM Hepes, pH 7.5/150 mM NaCl/0.1% Triton X-100/10% glycerol/200 $\mu$M orthovanadate, then twice again with the same buffer but with increasing salt concentration to 0.5 M NaCl, and finally with buffer containing 150 mM NaCl. The beads were boiled for 2 min. in 30 $\mu$l of Laemmli sample buffer (Laemmli, Nature 227:680-685, 1970). The immunoprecipitated protein was subjected to Western blot analysis (Ausubel, F.M. et al., eds., Current Protocols in Molecular Biology, Wiley, N.Y., Vol. 2, 1988) with an anti-phosphotyrosine antibody. To detect antibody binding, $^{125}$I-labeled protein A (New England Nuclear, Boston, Mass.; 500,000 cpm/ml in 10 mM Tris, pH 7.4/150 mM NaCl/1% bovine serum albumin) was added to the blot for 2 hr. and washed in 10 mM Tris, pH 7.4/150 mM NaCl/0.05% Triton X-100. The blot was then subjected to autoradiography for 2-5 days at room temperature.

As described in detail immediately above, serum-deprived BHK cells were stimulated with PDGF and extracted at various times in the presence of vanadate, and proteins phosphorylated on tyrosyl residues were immunoprecipitated with anti-phosphotyrosine antibody. The precipitated proteins were subjected to SDS/PAGE and then analyzed in a Western blot with a second anti-phosphotyrosine antibody. Autoradiography of the blot (Figure 2) revealed that 2 min. after PDGF stimulation, there was a dramatic increase in tyrosine phosphorylation in proteins of approximately 180, 140, 116, 92, and 60 kDa in the control cells. On the other hand, in cells overexpressing either the wild-type or truncated T-cell PTPase, there was a considerably lower level of phosphorylation in several protein bands, particularly those of 140, 116, and 60 kDa but not that of the 180-kDa protein assumed to be the PDGF receptor. The 116- ad 60-kDa proteins appeared to undergo dephosphorylation in extracts from control cells within this time period, suggesting that an endogenous PTPase was activated. Similar phosphoproteins were detected in phosphotyrosine immunoprecipitates from quiescent fibroblasts treated with PDGF or normal fibroblasts pretreated with vanadate.

In summary, the results show that the overexposed full-length 48-kDa T cell PTPase localizes to a particulate fraction that sediments at low speed. The enzyme is inactive *in vitro* toward RCM-lysozyme unless the fraction is pretreated with trypsin. This behavior can be ascribed to the 11-kDa carboxyl-terminal segment of the molecule. The enzyme is active *in vivo*. Despite this activity, there was no effect on BHK growth rate or gross morphology (by phase-contrast microscopy; however, there was an effect on the cytoskeleton) as compared with cells expressing the vector alone. It appears that the enzyme is highly regulated within the cells ad/or it may be partitioned into a compartment(s) where its activity would be restricted to specific substrates. The results also show that the overexpressed 37-kDa truncated form exhibits behavior different from the full-length enzyme. This truncated form is constitutively active and distributes evenly between particulate ad soluble fractions during purification. The BHk cells in which it was expressed grew at a reduced rate and displayed gross morphological changes. Therefore, it appears that the carboxyl-terminal segment is involved in the localization ad regulation of enzyme activity.

A hydrophobicity plot (Figure 3) indicates that this segment is essentially hydrophilic until approximately the last 20 residues, at which point the polypeptide chain becomes hydrophobic, with a hydrophobicity index approaching that of transmembrane segments or signal peptides. A similar distribution of hydrophobic and hydrophobic residues is also found in the carboxyl-terminal segments of low molecular mass human placenta ad rat brain PTPases, even though the primary structure of these segments is more variable than within their conserved 236-residue core structure. Sucrose density centrifugation gave no evidence for association of the T-cell PTPase enzyme with the plasma membrane. However, this PTPase might be interacting with other cellular components (nucleus, Golgi, or endoplasmic reticulum) or the cytoskeleton, since both high salt concentration and detergents are required for solubilization.

B. Baculovirus System

Wild type T cell cDNA and truncated T cell cDNA were introduced into Sf 9 (Spodoptera frugiperda) insect cells according to the method of Summers and Smith, Texas Agriculture Experimental Station Bulletin No. 1555, 1987. Briefly, the cDNA encoding T cell PTPase ad truncated T cell PTPase was inserted into the BamHI site that is 3' to the polyhedron promoter in the Baculoviral expression plasmid. The recombinant baculoviral DNA was cotransfected with the normal baculoviral genome into host Sf 9 insect cells (see Summers et al., *supra*). DNA was taken up by the cells, the recombinant plasmid and baculoviral DNA underwent genetic recombination *in vivo* and recombinant virus were generated. These viruses were selected by hybridization of the $^{32}$P-labeled T cell PTPase cDNA to insect cell lysates. The mRNA encoding PTPase protein was transcribed from the viral polyhedron promoter and the protein synthesized by the infected Sf 9 host cells. Approximately 30% of the total protein produced by the insect Sf 9 cells was due to the production of PTPase.

More specifically, open reading frames of the full-length 48-kDa T cell PTPase (TC.PTPase) and the 37-kDa truncated T cell PTPase (TCΔC11.PTPase) were isolated by a Thal/SspI digest (Examples 1 and 2, above) and cloned into the unique BamHI site of the plasmid pVL 941 (Luckow and Summers, Virology 170:31-39, 1989) after filling in the sticky ends with Klenow fragment and deoxynucleotides (Pharmacia). Correct orientation of insertion was confirmed by DNA sequence analysis (Sager et al., Proc. Natl. Acad. Sci. USA 74:5463-5467, 1977).

Sf9 cells were maintained in monolayer cultures as described (Summers and Smith, 1987). Cells were grown in Grace's Antheraea medium (Grace, Nature 195:788-789, 1962; Gibco, Grand Island, N.Y.)

supplemented with 3.3 g/L yeastolate (Difco Labs, Detroit, MI), 3.3 g/L lactalbumin hydrolysate (Difco labs, Detroit, MI), 10% fetal calf serum (Hink, Nature 226:446-467, 1970), and 100 U/mL penicillin, 100 $\mu$g/mL streptomycin and 0.25 $\mu$g/mL fungizone (Fungibact antibiotic mix, Irving Scientific, Santa Ana, Calif.). Cells were cotransfected with 1 $\mu$g Ac-NPV DNA and 2 $\mu$g plasmid (pAc-Tc.PTPase) as described by Summers and Smith, 1987. Purification of recombinant viruses was achieved by five rounds of serial end-point dilution and dot hybridization using $^{32}$P-labelled TC.PTPase cDNA as a probe. Purity of the final virus suspension was checked by hybridization with a $^{32}$P-labelled oligonucleotide probe; its sequence consists of nucleotides 37 to 66 of the Ac-NPV polyhedrin gene (Iddekinge et al., Virology 131:561-565, 1983), a portion missing in the pVL 941 expression vector. Extracts of Sf9 cells infected with pure recombinant virus do not hybridize with this oligonucleotide.

Cells were infected at a high (>3) multiplicity of infection and grown at 27°C. At different times post infection, cells were harvested by centrifugation for 5 min. at 5,000 x g. The following buffers were routinely used for extraction: (a) low salt buffer consisting of 25 mM imidazole pH 7.2, 2 mM EDTA, 0.1% 2-mercaptoethanol, 1 mM benzamidine, 0.002% phenylmethylsulfonyl fluoride, 2 $\mu$g/mL leupeptine, 1 $\mu$g/mL pepstatin, 5 kallikrein U/ml aprotinin; (b) low salt buffer containing 0.5% Triton X-100; (c) high salt buffer containing 0.6 M KCl and 0.5% Triton X-100 or 1% CHAPS.

Cells were suspended in low salt buffer (3 x $10^7$ cells/ml) and disrupted by 30 strokes in a Dounce homogenizer on ice. After 10 min. centrifugation at 10,000 x g, the pellet was resuspended in half of the original volume of low salt buffer containing 0.5% Triton X-100. The suspension was homogenized as previously described and centrifuged for 10 min. at 100,000 x g. Finally, the pellet was resuspended in high salt buffer containing 0.5% Triton X-100 or 1% CHAPS. The homogenate was again centrifuged for 10 min. at 100,000 x g; the supernatants were used for Western blot analysis, protein purification ad PTPase assays. For Western blot analysis, proteins were subjected to SDS-PAGE as described by Laemmli (Nature 227:680-685, 1970) and electrophoretically transferred to nitrocellulose. Rabbit antibody 8172 raised against a synthetic peptide derived from the amino terminal region of PTPase 1B (see Example 4 above) was used for detection in Western blots. Goat anti-rabbit IgG conjugated to alkaline phosphatase (Bio-Rad Laboratories, Richmond, Calif.) was used as a secondary antibody according to the manufacturer's instructions.

For purification of TC.PTPase, the Triton/KCl extract was made 20% in ammonium sulfate ad centrifuged for 10 min. at 10,000 x g. The precipitate was resuspended in 200 $\mu$L high salt buffer containing 0.5% Triton X-100 and applied to a Superose 12 FPLC column equilibrated in the same buffer. For purification of the C-terminal truncated enzyme, the low salt buffer extract was directly applied to a Sephadex G75 superfine column (2.6 cm x 82.5 cm, flow rate ca. 10 mL/h). Peak fractions were made 20% in glycerol and frozen at -70°C.

PTPase activity was measured as described in Example 3 above, except in some assays phosphorylated myelin basic protein (MBP) was used in saturating concentrations as substrate instead of tyrosyl phosphorylated RCM-lysozyme (RCML). Trypsin treatment was carried out by incubating the PTPase (<0.25 U/ml) in 40 $\mu$l of assay buffer with 1 $\mu$g of trypsin for 5 min. at 30°C. Trypsin was inhibited by addition of 6 $\mu$g of lima bean trypsin inhibitor, and the phosphatase reaction was started by addition of substrate.

The internal kinase domain of the epidermal growth factor receptor (EGFR) as expressed in the baculovirus system (Hsu et al., Cell Growth Differ. 1:191-200, 1990) was autophosphorylated in 20 mM HEPES pH 7.5, 0.1% 2-mercaptoethanol, 5% glycerol, 1 $\mu$g/mL pepstatin, 5 kallikrein U/mL aprotinin, 2 $\mu$g/mL leupeptin, 5 mM manganese acetate and 0.1 mM ATP (2.2 x $10^5$ cpm/pmol) for 10 min. at 30°C. The kinase reaction was stopped by adding EDTA to a concentration of 10 mM. The autophosphorylated receptor (80 ng) was then incubated either with buffer or with 100 ng of TC.PTPase or TC$\Delta$C11.PTPase, respectively, for 20 min. at 30°C. Sample buffer was added and the reaction run onto a 7.5% SDS-PAGE. The gel was dried and autoradiographed.

As described above, Sf9 cells were cotransfected with pAc-TC.PTPase plasmid DNA and Ac-NPV wild type DNA. At different times post infection, cells were harvested and lysed; the lysates were subjected to SDS-PAGE and immunoblot analysis. Uninfected cells or cells infected with wild type (wt) Ac-NPV served as controls. The expression level (observed by Western blot analysis) of both TC.PTPase and TC$\Delta$C11.PTPase increased steadily from day 2 to 5; after 5 days, the majority of cells had lysed due to the viral infection. Additional bands of higher Mr appeared after 3 days, probably due to some post-translational modification. Limited trypsinolysis of both proteins gave rise to a tryptic fragment of approximately 33 kDa. Since antibody 8172 recognizes a sequence near the N-terminus of the enzyme, the main tryptic cleavage must have occurred in the C-terminal region of the protein. No cross-reacting material was detected in uninfected cells or in cells infected with the wt virus. The increase PTPase activity in extracts from infected Sf9 cells paralleled the increased level of expression as observed by Western blot analysis. Substantial activity of the TC.PTPase towards RCML could be seen only following limited trypsinolysis of the enzyme.

The cells were extracted first in low salt buffer and the suspension centrifuged. The pellet was re-extracted with buffer containing 0.5% Triton X-100; this suspension was recentrifuged, and the second pellet was extracted with the same Triton buffer but in the presence of 0.6 M KCl. The full-length enzyme could be solubilized only by such a combination of salt and detergent; in contrast, the truncated form was readily soluble in aqueous buffer.

The TC.PTPase from Triton/KCl extracts was precipitated by adding ammonium sulfate to 20% saturation. The suspension was centrifuged and the pellet solubilized in Triton/KCl buffer. Although the recovery was only ca. 50%, this step was necessary to concentrate the enzyme. The protein solution was applied to a Superose 12 FPLC column. TC.PTPase eluted as one major peak of activity with a trailing shoulder. When fractions from both (18-20 ad 21-24) were collected, the material in each pool resulted in the same bad pattern on SDS-PAGE displaying a doublet at 48 kDa ad a faint band at 40 kDa. Due to contaminants, the specific activity of the material in the trailing edge was approximately half that of the forward peak. Both fractions eluted at molecular weights (220 k and 160 k, respectively) higher than expected for the monomeric molecule (48 k), implying aggregation, insertion into detergent micelles or asymmetry of the molecule.

Aggregation of the protein could not be demonstrated directly. Assuming that the activation of the enzyme by polyamines or MBP resulted from disaggregation, the full-length PTPase was chromatographed on the same FPLC Superose 12 column after equilibrating the column in 2 mM spermine or after preincubation with a ten-fold molar excess of unphosphorylated MBP. No shift in the elution pattern was observed. Furthermore, attempts at cross-linking with dimethylsuberimidate revealed no protein species with a Mr higher than 4 k on SDS-PAGE, whereas cross-linking of hemoglobin as a control under the same conditions was successful.

To test the possible influence of the detergent, chromatography was also carried out in CHAPS which forms smaller micelles (ca. 5 kDa than Triton X-100 (ca. 90 kDa). However, here again, the enzyme eluted at a Mr higher than expected (ca. 170 k).

All three band visible in SDS-PAGE correspond to different forms of the TC.PTPase since they are recognized by antibody 8172 ad two other antibodies directed against different segments of the T-cell PTPase (residues 342-357 and 369-381). Further characterization of the enzyme was performed on material obtained from peak fractions 19 and 20.

Since 90% of TCΔC11.PTPase distributed in the aqueous buffer, the extract could be applied directly to a Sephadex G75 superfine column. In contrast to the full-length protein, TCΔC11.PTPase eluted at its expected molecular weight. Table 2 summarizes the purification of both forms of the T-cell PTPase. These could be stored for months in the presence of 20% glycerol at -70°C without significant loss of activity.

TABLE 2

| Purification of expressed TC.PTPase (A) and TCΔC11.PTPase (B) | | | | | | |
|---|---|---|---|---|---|---|
| A | volume [ml] | total activity[a] [units] | protein [mg] | specific activity[a] [units/mg] | purification [fold] | yield [%] |
| Triton/KCl extract | 1.0 | 12,000 | 4.6 | 2.650 | 1 | 100 |
| (NH$_4$)$_2$SO$_4$ precipitate | 0.2 | 6,400 | 2.3 | 2,850 | 1 | 53 |
| Superose 12 peak | 0.5 | 3,250 | 0.3 | 10,700 | 4 | 27 |
| B | volume [ml] | total activity[b] [units] | protein [mg] | specific activity[b] [units/mg] | purification [fold] | yield [%] |
| extract | 3.2 | 14,900 | 4.65 | 3,200 | 1 | 100 |
| G75 Sephadex peak | 9.0 | 7,200 | 0.24 | 30,200 | 9 | 48 |

[a] activities determined with MBP as substrate in the presence of 5 mM EDTA
[b] activities determined with RCML as substrate in the presence of 5 mM EDTA

Both forms of the T-cell PTPase were totally specific for phosphotyrosyl residues showing no activity towards MBP or histones phosphorylated by the cAMP-dependent protein kinase. The truncated form of the PTPase displayed a specific activity of 26,000 U/mg toward tyrosyl phosphorylated RCML. By contrast, the full-length enzyme was far less active (850 U/mg) suggesting that enzyme activity is repressed by the C-terminal segment (Table 3) Both limited trypsinolysis and truncation of the molecule by introduction of a premature stop codon led to a 30-fold increase in activity toward RCML. The activity of the full-length enzyme depended greatly on the nature of the substrate. In the presence of phosphorylated MBP, it displayed a specific activity of 10,300 U/mg as compared to 4,700 U/mg only for the truncated form. These data suggest that MBP interacts with the C-terminal segment, resulting in an activation of the enzyme. Both forms of the T-cell PTPase readily dephosphorylated the soluble kinase domain of the EGFR following its autophosphorylation.

TABLE 3

| Enzymatic Properties of PTPase Forms | | | | |
|---|---|---|---|---|
| Substrate: | RCML | | MBP | |
| | specific activity[a] [units/mg] | $K_m$ [nM] | specific activity[a] [units/mg] | Km [nM] |
| TC.PTPase | 850 ±170 | 200 | 10,300 ±1,300 | 500 |
| after trypsinolysis | 23,300 ±3,800 | n.d. | 3,600 ±1,000 | n.d. |
| TC$\Delta$C11.PTPase | 26,000 ±3,000 | 50 | 4,700 ±500 | 1250 |
| after trysinolysis | 15,300 ±2,600 | n.d. | 1,900 ±200 | n.d. |

[a] average ad standard deviation for three separate preparations measured under conditions of substrate saturation in the presence of 5 mM EDTA

Both forms of the T cell enzyme were inhibited by micromolar concentrations of the classical inhibitors vanadate (Swarup et al., Biochem. Biophys. Res. Commun. 107:1104-1100, 1982), molybdate ad $Zn^{2+}$ - (Brautigan et al., J. Biol. Chem. 256:6519-6522, 1981) (Table 4). Calcium and magnesium were essentially without effect. Both forms of the T cell enzyme were inhibited by nanomolar concentrations of polyanions; this effect was only observed with RCML as substrate. The enzyme was activated by EDTA, but not as potently as PTPase 1B. The full-length enzyme was markedly activated by polycationic compounds (up to sevenfold by spermine and up to threefold by unphosphorylated MBP) only when negatively charged RCML was used as the substrate. By contrast, activation of the truncated form of the enzyme by these compounds was 30% at most, suggesting that the polycationic molecules interact with the C-terminal region.

## TABLE 4

### Effectors of PTPase Activity

Activity is expressed as the percentage of phosphate released relative to control in which the effector was omitted. All assays were performed in duplicate in the presence of 5 $\mu$M substrate.

| Enzyme form: | TC.PTPase | | TC$\Delta$C11.PTPase | |
|---|---|---|---|---|
| Substrate: | RCML | MBP | RCML | MBP |
| effector | | | | |
| none | 100 | 100 | 100 | 100 |
| 100 $\mu$M vanadate | 0 | 0 | 2 | 1 |
| 10 $\mu$M molybdate | 0 | 1 | 2 | 2 |
| 100 $\mu$M Zn$^{++}$ | 33 | 101 | 15 | 66 |
| 0.01 $\mu$M heparin | 96 | 120 | 58 | 102 |
| 1 $\mu$M heparin | 8 | 108 | 8 | 75 |
| 10 $\mu$M heparin | 5 | 73 | 1 | 90 |
| 0.01 $\mu$M poly Glu:Tyr 4:1 | 66 | 119 | 70 | 102 |
| 1 $\mu$M poly Glu:Tyr 4:1 | 18 | 133 | 17 | 104 |
| 10 $\mu$M poly Glu:Tyr 4:1 | 6 | 58 | 11 | 127 |
| 5 mM EDTA | 124 | 137 | 170 | 150 |
| 2 mM spermine | 727 | 62 | 110 | 97 |
| 2 mM spermidine | 249 | 43 | 132 | 108 |
| 1 $\mu$M unphosphor. MBP | 122 | 62 | 110 | 97 |
| 10 $\mu$M unphosphor. MBP | 297 | 43 | 132 | 108 |
| 50 $\mu$M unphosphor. MBP | 279 | 15 | 10 | 66 |

In addition to the method described above, U.S. Patent Nos. 4,879,236; 4,870,023; and 4,745,051 (herein incorporated by reference) disclose methods for producing recombinant baculoviruses and their infection of insect cells.

C. Retroviruses

Wild type T cell PTPase (48 kDa) cDNA or c-terminal truncated PTPase cDNA are introduced into cells according to the method of Miller and Rosman (BioTechniques 7:980-990, 1989). The cDNAs of interest are ligated into the vector LXSN (kindly provided by Dr. Miller, Fred Hutchinson Cancer Research Center) and transfected by the calcium phosphate precipitation method into a retroviral packaging cell line (PE501) (provided by Dr. Miller). The cells which contain the DNA are resistant to the drug G418 and thus are selected for their ability to grow in the presence of 1.5 mg/ml of the drug in the media. Individual colonies are isolated and their supernatants containing recombinant retroviruses are saved.

A number of cell lines are appropriate to test whether the retroviruses are infectious and are producing the PTPases *in vivo*. These include NIH 3T3, NIH Swiss D1, NIH 3T3 transformed by v-src, Rat 2, and Rat 2 transformed by v-fms. Approximately $10^5$ cells are plated, infected with 100 $\mu$l of retroviral supernatant from each of the colonies of retroviral producing cell lines. Positive infection is tested by selection in G418 media. Finally, any G418-resistant cells are analyzed for expression of either the 48 kDa or the c-terminal truncated PTPase by immunoprecipitation with a specific anti - T cell PTPase antibody (e.g., the antisera of Example 4).

EXAMPLE 6

REVERSION OF MALIGNANT TRANSFORMATION OF BHK CELLS BY TRANSFECTION WITH T CELL cDNA

DNA encoding a modified form of the 48 kDa form of the T cell PTPase was introduced into Baby Hamster Kidney (BHK) cells. The modification of the cDNA is a deletion of an 11 kDa carboxy-terminal extension which renders the enzyme very active with a specific activity of 40,000 units/mg *in vitro*. The expression of this modified PTPase is not toxic to the BHK cells. In addition, BHK cells expressing this enzyme exhibited an altered phenotype in that they were no longer transformed.

The nature of the transformation of baby hamster kidney (BHK) cells is unknown. Two standard techniques for determining oncogenicity are: (1) to grow cells in soft agar demonstrating that the cells are no longer contact inhibited, and (2) to inject cells into nude mice (i.e., mice which are lacking a thymus and so cannot reject the cells) and look for tumor growth at the point of injection. These tests were positive for BHK cells, BHK cells transfected with an expression vector, BHK cells transfected with the 48 kDa form of the T cell PTPase, but negative for the cells expressing high levels of the c-terminal truncated form of the T cell PTPase.

For example, the control cells showed colonies in soft agar in two weeks which were much greater than 2 $\mu$m in diameter whereas all the colonies containing cells expressing the truncated PTPase were less than 2 $\mu$m. This was true for cells grown up to one month in soft agar. The volumes of the tumors formed after one month in nude mice which had been injected with the control cells, i.e., 2.5 x $10^6$ cells expressing either the vector or the 48 kDa enzyme, were 1.5 ml and 2.1 ml, respectively, and these large tumors undergo angiogenesis. The volumes in nude mice injected with 5 x $10^6$ cells expressing a c-terminal truncated form of the T cell PTPase ranged from 0.21-0.38 ml, and these small growths do not undergo angiogenesis.

From the foregoing, it will be evident that, although specific embodiments of the invention have been described herein for purposes of illustration, various modifications may be made without deviating from the spirit and scope of the invention.

**Claims**
**Claims for the following Contracting States : AT, BE, CH, DE, DK, FR, GB, IT, LI, LU, NL, SE**

1. A DNA molecule encoding a carboxyl-terminus-truncated non-receptor-linked protein tyrosine phosphatase, wherein said protein tyrosine phosphatase consists essentially of the amino acid sequence of Figure 1 from methionine, amino acid 1, to an amino acid positioned between leucine, amino acid 297, and arginine, amino acid 317.

2. A DNA molecule encoding a carboxyl-terminus-truncated non-receptor-linked protein tyrosine phosphatase, wherein said protein tyrosine phosphatase consists essentially of the amino acid sequence of Figure 1 from methionine, amino acid 1, to arginine, amino acid 317.

3. A DNA molecule encoding a carboxyl-terminus-truncated non-receptor-linked protein tyrosine phosphatase, wherein said protein tyrosine phosphatase consists essentially of an amino acid sequence of between 297 to 320 amino acid residues, and wherein said amino acid sequence contains a portion of similar length to and having at least approximately 80% sequence similarity with the amino acid sequence of Figure 1 from asparagine, amino acid 42, to glutamic acid, amino acid 274.

4. A DNA molecule encoding a carboxyl-terminus-truncated non-receptor-linked protein tyrosine phosphatase, wherein said protein tyrosine phosphatase consists essentially of the amino acid sequence of Figure 1 from methionine, amino acid 1, to an amino acid positioned between glutamic acid, amino acid 376, to serine, amino acid 396.

5. A gene transfer vehicle capable of infecting malignant cells, said gene transfer vehicle carrying a DNA construct comprising a DNA molecule encoding a carboxyl-terminus-truncated non-receptor-linked protein tyrosine phosphatase.

6. The gene transfer vehicle according to Claim 5 wherein said DNA molecule encodes a carboxyl-terminus-truncated non-receptor-linked protein tyrosine phosphatase, wherein said protein tyrosine phosphatase comprises an amino acid sequence of between 297 to 321 amino acid residues, and wherein said amino acid sequence contains a portion of similar length to and having at least approximately 80% sequence similarity with the amino acid sequence of Figure 1 from asparagine, amino acid 42, to glutamic acid, amino acid 274.

7. A gene transfer vehicle capable of infecting malignant cells, said gene transfer vehicle carrying a DNA construct, comprising a DNA molecule according to any one of Claims 1, 2 or 4.

8. A gene transfer vehicle according to any one of Claims 5 to 7 wherein said gene transfer vehicle comprises a recombinant retrovirus or a recombinant vaccinia virus.

9. A gene transfer vehicle according to any one of Claims 5 to 8, for use within a method for treating a malignancy in a warm-blooded animal, wherein a protein tyrosine kinase is associated with the malignancy.

**Claims for the following Contracting States : ES GR**

1. A method of producing a DNA molecule which method comprises producing a DNA molecule encoding a carboxyl-terminus-truncated non-receptor-linked protein tyrosine phosphatase, wherein said protein tyrosine phosphatase consists essentially of the amino acid sequence of Figure 1 from methionine, amino acid 1, to an amino acid positioned between leucine, amino acid 297, and arginine, amino acid 317.

2. A method of producing a DNA molecule which method comprises producing a DNA molecule encoding a carboxyl-terminus-truncated non-receptor-linked protein tyrosine phosphatase, wherein said protein tyrosine phosphatase consists essentially of the amino acid sequence of Figure 1 from methionine, amino acid 1, to arginine, amino acid 317.

3. A method of producing a DNA molecule which method comprises producing a DNA molecule encoding a carboxyl-terminus-truncated non-receptor-linked protein tyrosine phosphatase, wherein said protein tyrosine phosphatase consists essentially of an amino acid sequence of between 297 to 320 amino acid residues, and wherein said amino acid sequence contains a portion of similar length to and having at least approximately 80% sequence similarity with the amino acid sequence of Figure 1 from asparagine, amino acid 42, to glutamic acid, amino acid 274.

4. A method of producing a DNA molecule which method comprises producing a DNA molecule encoding a carboxyl-terminus-truncated non-receptor-linked protein tyrosine phosphatase, wherein said protein tyrosine phosphatase consists essentially of the amino acid sequence of Figure 1 from methionine, amino acid 1, to an amino acid positioned between glutamic acid, amino acid 376, to serine, amino acid 396.

5. A method of producing a gene transfer vehicle capable of infecting malignant cells, which method comprises producing a gene transfer vehicle carrying a DNA construct comprising a DNA molecule encoding a carboxyl-terminus-truncated non-rector-linked protein tyrosine phosphatase.

6. The method according to Claim 5 wherein said DNA molecule encodes a carboxyl-terminus-truncated non-receptor-linked protein tyrosine phosphatase, wherein said protein tyrosine phosphatase comprises an amino acid sequence of between 297 to 321 amino acid residues, and wherein said amino acid sequence contains a portion of similar length to and having at least approximately 80% sequence similarity with the amino acid sequence of Figure 1 from asparagine, amino acid 42, to glutamic acid, amino acid 274.

7. A method of producing a gene transfer vehicle capable of infecting malignant cells, which method comprises producing gene transfer vehicle carrying a DNA construct comprising a DNA molecule producible according to any one of Claims 1, 2 or 4.

8. A method according to any one of Claims 5 to 7 wherein said gene transfer vehicle comprises a recombinant retrovirus or a recombinant vaccinia virus.

9. A gene transfer vehicle capable of infecting malignant cells, said gene transfer vehicle carrying DNA construct comprising a DNA molecule encoding a non-receptor-linked protein tyrosine phosphatase for use within a method for treating a malignancy in a warm-blooded animal, wherein a protein tyrosine kinase is associated with the malignancy.

**Patentansprüche**
**Patentansprüche für folgende Vertragsstaaten : AT, BE, CH, DE, DK, FR, GB, IT, LI, LU, NL, SE**

1. Ein DNA-Molekül, das eine Carboxy-Terminus-gestutzte, nicht-rezeptorverknüpfte Proteintyrosinphosphatase kodiert, wobei besagte Proteintyrosinphosphatase im wesentlichen aus der Aminosäuresequenz von Figur 1 von Methionin, Aminosäure 1, bis zu einer Aminosäure, die zwischen Leucin, Aminosäure 297, und Arginin, Aminosäure 317, angeordnet ist, besteht.

2. Ein DNA-Molekül, das eine Carboxy-Terminus-gestutzte, nicht-rezeptorverknüpfte Proteintyrosinphosphatase kodiert, wobei besagte Proteintyrosinphosphatase im wesentlichen aus der Aminosäuresequenz von Figur 1 von Methionin, Aminosäure 1, bis Arginin, Aminosäure 317, besteht.

3. Ein DNA-Molekül, das eine Carboxy-Terminus-gestutzte, nicht-rezeptorverknüpfte Proteintyrosinphosphatase kodiert, wobei besagte Proteintyrosinphosphatase im wesentlichen aus einer Aminosäuresequenz mit zwischen 297 bis 320 Aminosäureresten besteht und wobei besagte Aminosäuresequenz einen Abschnitt mit ähnlicher Länge und mit wenigstens ungefähr 80% Sequenzähnlichkeit mit der Aminosäuresequenz von Figur 1 von Asparagin, Aminosäure 42, bis Glutaminsäure, Aminosäure 274, enthält.

4. Ein DNA-Molekül, das eine Carboxy-Terminus-gestutzte, nicht-rezeptorverknüpfte Proteintyrosinphosphatase kodiert, wobei besagte Proteintyrosinphosphatase im wesentlichen aus der Aminosäuresequenz von Figur 1 von Methionin, Aminosäure 1, bis zu einer Aminosäure, die zwischen Glutaminsäure, Aminosäure 376, bis Serin, Aminosäure 396, angeordnet ist, besteht.

5. Ein Gen-Transfervehikel, das in der Lage ist, bösartige Zellen zu infizieren, wobei besagtes Gen-Transfervehikel ein DNA-Konstrukt trägt, das ein DNA-Molekül umfaßt, das eine Carboxy-Terminusgestutzte, nicht-rezeptorverknüpfte Proteintyrosinphosphatase kodiert.

6. Das Gen-Transfervehikel nach Anspruch 5, wobei besagtes DNA-Molekül eine Carboxy-Terminusgestutzte, nicht-rezeptorverknüpfte Proteintyrosinphosphatase kodiert, wobei besagte Proteintyrosinphosphatase eine Aminosäuresequenz mit zwischen 297 bis 321 Aminosäureresten umfaßt und wobei besagte Aminosäuresequenz einen Abschnitt mit ähnlicher Länge und mit wenigstens ungefähr 80% Sequenzähnlichkeit mit der Aminosäuresequenz von Figur 1 von Asparagin, Aminosäure 42, bis Glutaminsäure, Aminosäure 274, enthält.

7. Ein Gen-Transfervehikel, das in der Lage ist, bösartige Zellen zu infizieren, wobei besagtes Gen-Transfervehikel ein DNA-Konstrukt trägt, das ein DNA-Molekül nach einem der Ansprüche 1, 2 oder 4 umfaßt.

8. Ein Gen-Transfervehikel nach einem der Ansprüche 5 bis 7, wobei besagtes Gen-Transfervehikel ein rekombinantes Retrovirus oder ein rekombinantes Vaccinia-Virus umfaßt.

9. Ein Gen-Transfervehikel nach einem der Ansprüche 5 bis 8, zur Verwendung in einer Methode zur Behandlung einer bösartigen Entartung in einem Warmblüter, wobei eine Proteintyrosinkinase mit der bösartigen Entartung im Zusammenhang steht.

**Patentansprüche für folgende Vertragsstaaten : ES GR**

1. Ein Verfahren zur Herstellung eines DNA-Moleküls, wobei das Verfahren die Herstellung eines DNA-Moleküls umfaßt, das eine Carboxy-Terminus-gestutzte, nicht-rezeptorverknüpfte Proteintyrosinphosphatase kodiert, wobei besagte Proteintyrosinphosphatase im wesentlichen aus der Aminosäuresequenz von Figur 1 von Methionin, Aminosäure 1, bis zu einer Aminosäure, die zwischen Leucin, Aminosäure 297, und Arginin, Aminosäure 317, angeordnet ist, besteht.

2. Ein Verfahren zur Herstellung eines DNA-Moleküls, wobei das Verfahren die Herstellung eines DNA-Moleküls umfaßt, das eine Carboxy-Terminus-gestutzte, nicht-rezeptorverknüpfte Proteintyrosinphosphatase kodiert, wobei besagte Proteintyrosinphosphatase im wesentlichen aus der Aminosäuresequenz von Figur 1 von Methionin, Aminosäure 1, bis Arginin, Aminosäure 317, besteht.

3. Ein Verfahren zur Herstellung eines DNA-Moleküls, wobei das Verfahren die Herstellung eines DNA-Moleküls umfaßt, das eine Carboxy-Terminus-gestutzte, nicht-rezeptorverknüpfte Proteintyrosinphosphatase kodiert, wobei besagte Proteintyrosinphosphatase im wesentlichen aus einer Aminosäuresequenz mit zwischen 297 bis 320 Aminosäureresten besteht und wobei besagte Aminosäuresequenz einen Abschnitt mit ähnlicher Länge und mit wenigstens ungefähr 80% Sequenzähnlichkeit mit der Aminosäuresequenz von Figur 1 von Asparagin, Aminosäure 42, bis Glutaminsäure, Aminosäure 274, enthält.

4. Ein Verfahren zur Herstellung eines DNA-Moleküls, wobei das Verfahren die Herstellung eines DNA-Moleküls umfaßt, das eine Carboxy-Terminus-gestutzte, nicht-rezeptorverknüpfte Proteintyrosinphosphatase kodiert, wobei besagte Proteintyrosinphosphatase im wesentlichen aus der Aminosäuresequenz von Figur 1 von Methionin, Aminosäure 1, bis zu einer Aminosäure, die zwischen Glutaminsäure, Aminosäure 376, bis Serin, Aminosäure 396, angeordnet ist, besteht.

5. Ein Verfahren zur Herstellung eines Gen-Transfervehikels, das in der Lage ist, bösartige Zellen zu infizieren, wobei das Verfahren die Herstellung eines Gen-Transfervehikels umfaßt, das ein DNA-Konstrukt trägt, das ein DNA-Molekül umfaßt, das eine Carboxy-Terminus-gestutzte, nicht-rezeptorverknüpfte Proteintyrosinphosphatase kodiert.

6. Das Verfahren nach Anspruch 5, wobei besagtes DNA-Molekül eine Carboxy-Terminus-gestutzte, nicht-rezeptorverknüpfte Proteintyrosinphosphatase kodiert, wobei besagte Proteintyrosinphosphatase eine Aminosäuresequenz mit zwischen 297 bis 321 Aminosäureresten umfaßt und wobei besagte Aminosäuresequenz einen Abschnitt mit ähnlicher Länge und mit wenigstens ungefähr 80% Sequenzähnlichkeit mit der Aminosäuresequenz von Figur 1 von Asparagin, Aminosäure 42, bis Glutaminsäure, Aminosäure 274, enthält.

7. Ein Verfahren zur Herstellung eines Gen-Transfervehikels, das in der Lage ist, bösartige Zellen zu infizieren, wobei das Verfahren die Herstellung eines Gen-Transfervehikels umfaßt, das ein DNA-Konstrukt trägt, das ein DNA-Molekül umfaßt, das nach einem der Ansprüche 1, 2 oder 4 herstellbar ist.

8. Ein Verfahren nach einem der Ansprüche 5 bis 7, wobei besagtes Gen-Transfervehikel ein rekombinantes Retrovirus oder ein rekombinantes Vaccinia-Virus umfaßt.

9. Ein Gen-Transfervehikel, das in der Lage ist, bösartige Zellen zu infizieren, wobei besagtes Gen-Transfervehikel ein DNA-Konstrukt trägt, das ein DNA-Molekül umfaßt, das eine nicht-rezeptorverknüpfte Proteintyrosinphosphatase kodiert, zur Verwendung in einer Methode zur Behandlung einer bösartigen Entartung in einem Warmblüter, wobei eine Proteintyrosinkinase mit der bösartigen Entartung in Zusammenhang steht.

**Revendications**

**Revendications pour les Etats contractants suivants : AT, BE, CH, DE, DK, FR, GB, IT, LI, LU, NL, SE**

1.  Molécule d'ADN codant pour une protéine tyrosine phosphatase non liée à un récepteur, tronquée à l'extrémité carboxy-terminale, caractérisée en ce que ladite protéine tyrosine phosphatase est constituée essentiellement de la séquence d'acides aminés de la figure 1, de la méthionine, acide aminé 1, à un acide aminé situé entre la leucine, acide aminé 297, et l'arginine, acide aminé 317.

2.  Molécule d'ADN codant pour une protéine tyrosine phosphatase non liée à un récepteur, tronquée à l'extrémité carboxy-terminale, caractérisée en ce que ladite protéine tyrosine phosphatase est constituée essentiellement de la séquence d'acides aminés de la figure 1, de la méthionine, acide aminé 1, à l'arginine, acide aminé 317.

3.  Molécule d'ADN codant pour une protéine tyrosine phosphatase non liée à un récepteur, tronquée à l'extrémité carboxy-terminale, caractérisée en ce que ladite protéine tyrosine phosphatase est constituée essentiellement d'une séquence d'acides aminés ayant entre 297 et 320 résidus d'acides aminés, et en ce que ladite séquence d'acides aminés contient une partie de longueur similaire à et présentant une similitude de séquence d'au moins approximativement 80% avec la séquence d'acides aminés de la figure 1, de l'asparagine, acide aminé 42, à l'acide glutamique, acide aminé 274.

4.  Molécule d'ADN codant pour une protéine tyrosine phosphatase non liée à un récepteur, tronquée à l'extrémité carboxy-terminale, caractérisée en ce que ladite protéine tyrosine phosphatase est constituée essentiellement de la séquence d'acides aminés de la figure 1, de la méthionine, acide aminé 1, à un acide aminé situé entre l'acide glutamique, acide aminé 376, et la sérine, acide aminé 396.

5.  Véhicule de transfert de gène capable d'infecter des cellules malignes, ledit véhicule de transfert de gène portant une construction d'ADN comprenant une molécule d'ADN codant pour une protéine tyrosine phosphatase non liée à un récepteur, tronquée à l'extrémité carboxy-terminale.

6.  Véhicule de transfert de gène selon la revendication 5, caractérisé en ce que ladite molécule d'ADN code pour une protéine tyrosine phosphatase non liée à un récepteur, tronquée à l'extrémité carboxy-terminale, en ce que ladite protéine tyrosine phosphatase comprend une séquence d'acides aminés ayant entre 297 et 321 résidus d'acides aminés, et en ce que ladite séquence d'acides aminés contient une partie de longueur similaire à et présentant une similitude de séquence d'au moins approximativement 80% avec la séquence d'acides aminés de la figure 1, de l'asparagine, acide aminé 42, à l'acide glutamique, acide aminé 274.

7.  Véhicule de transfert de gène capable d'infecter des cellules malignes, ledit véhicule de transfert de gène portant une construction d'ADN, comprenant une molécule d'ADN selon l'une quelconque des revendications 1, 2 ou 4.

8.  Véhicule de transfert de gène selon l'une quelconque des revendications 5 à 7, caractérisé en ce que ledit véhicule de transfert de gène comprend un rétrovirus recombinant ou un virus de la vaccine recombinant.

9.  Véhicule de transfert de gène selon l'une quelconque des revendications 5 à 8, destiné à l'utilisation dans un procédé de traitement d'une tumeur maligne chez un animal à sang chaud, caractérisé en ce qu'une protéine tyrosine kinase est associée à la tumeur maligne.

**Revendications pour les Etats contractants suivants : ES GR**

1.  Procédé de production d'une molécule d'ADN, lequel procédé comprend la production d'une molécule d'ADN codant pour une protéine tyrosine phosphatase non liée à un récepteur, tronquée à l'extrémité carboxy-terminale, caractérisée en ce que ladite protéine tyrosine phosphatase est constituée essentiellement de la séquence d'acides aminés de la figure 1, de la méthionine, acide aminé 1, à un acide aminé situé entre la leucine, acide aminé 297, et l'arginine, acide aminé 317.

22

2. Procédé de production d'une molécule d'ADN, lequel procédé comprend la production d'une molécule d'ADN codant pour une protéine tyrosine phosphatase non liée à un récepteur, tronquée à l'extrémité carboxy-terminale, caractérisée en ce que ladite protéine tyrosine phosphatase est constituée essentiellement de la séquence d'acides aminés de la figure 1, de la méthionine, acide aminé 1, à l'arginine, acide aminé 317.

3. Procédé de production d'une molécule d'ADN, lequel procédé comprend la production d'une molécule d'ADN codant pour une protéine tyrosine phosphatase non liée à un récepteur, tronquée à l'extrémité carboxy-terminale, caractérisée en ce que ladite protéine tyrosine phosphatase est constituée essentiellement d'une séquence d'acides aminés ayant entre 297 et 320 résidus d'acides aminés, et en ce que ladite séquence d'acides aminés contient une partie de longueur similaire à et présentant une similitude de séquence d'au moins approximativement 80% avec la séquence d'acides aminés de la figure 1, de l'asparagine, acide aminé 42, à l'acide glutamique, acide aminé 274.

4. Procédé de production d'une molécule d'ADN, lequel procédé comprend la production d'une molécule d'ADN codant pour une protéine tyrosine phosphatase non liée à un récepteur, tronquée à l'extrémité carboxy-terminale, caractérisée en ce que ladite protéine tyrosine phosphatase est constituée essentiellement de la séquence d'acides aminés de la figure 1, de la méthionine, acide aminé 1, à un acide aminé situé entre l'acide glutamique, acide aminé 376, et la sérine, acide aminé 396.

5. Procédé de production d'un véhicule de transfert de gène capable d'infecter des cellules malignes, lequel procédé comprend la production d'un véhicule de transfert de gène portant une construction d'ADN comprenant une molécule d'ADN codant pour une protéine tyrosine phosphatase non liée à un récepteur, tronquée à l'extrémité carboxy-terminale.

6. Procédé selon la revendication 5, caractérisé en ce que ladite molécule d'ADN code pour une protéine tyrosine phosphatase non liée à un récepteur, tronquée à l'extrémité carboxy-terminale, en ce que ladite protéine tyrosine phosphatase comprend une séquence d'acides aminés ayant entre 297 et 321 résidus d'acides aminés, et en ce que ladite séquence d'acides aminés contient une partie de longueur similaire à et présentant une similitude de séquence d'au moins approximativement 80% avec la séquence d'acides aminés de la figure 1, de l'asparagine, acide aminé 42, à l',acide glutamique, acide aminé 274.

7. Procédé de production d'un véhicule de transfert de gène capable d'infecter des cellules malignes, lequel procédé comprend la production d'un véhicule de transfert de gène portant une construction d'ADN, comprenant une molécule d'ADN productible selon l'une quelconque des revendications 1, 2 ou 4.

8. Procédé selon l'une quelconque des revendications 5 à 7, caractérisé en ce que ledit véhicule de transfert de gène comprend un rétrovirus recombinant ou un virus de la vaccine recombinant.

9. Véhicule de transfert de gène capable d'infecter des cellules malignes, ledit véhicule de transfert de gène portant une construction d'ADN, comprenant une molécule d'ADN codant pour une protéine tyrosine phosphatase non liée à un récepteur, destiné à l'utilisation dans un procédé de traitement d'une tumeur maligne chez un animal à sang chaud, caractérisé en ce qu'une protéine tyrosine kinase est associée à la tumeur maligne.

# FIG. 1

```
1   GGG GGG CCT GAG CCT CTC CGC CGG CGC AGG CTC TGC TCG CGC CAG


46  CTC GCT CCC GCA GCC ATG CCC ACC ACC ATC GAG CGG GAG TTC GAA
                    Met Pro Thr Thr Ile Glu Arg Glu Phe Glu 10


91  GAG TTG GAT ACT CAG CGT CGC TGG CAG CCG CTG TAC TTG GAA ATT
    Glu Leu Asp Thr Gln Arg Arg Trp Gln Pro Leu Tyr Leu Glu Ile 25


136 CGA AAT GAG TCC CAT GAC TAT CCT CAT AGA GTG GCC AAG TTT CCA
    Arg Asn Glu Ser His Asp Tyr Pro His Arg Val Ala Lys Phe Pro 40


181 GAA AAC AGA AAT CGA AAC AGA TAC AGA GAT GTA AGC CCA TAT GAT
    Glu Asn Arg Asn Arg Asn Arg Tyr Arg Asp Val Ser Pro Tyr Asp 55
        ↑

226 CAC AGT CGT GTT AAA CTG CAA AAT GCT GAG AAT GAT TAT ATT AAT
    His Ser Arg Val Lys Leu Gln Asn Ala Glu Asn Asp Tyr Ile Asn 70


271 GCC AGT TTA GTT GAC ATA GAA GAG GCA CAA AGG AGT TAC ATC TTA
    Ala Ser Leu Val Asp Ile Glu Glu Ala Gln Arg Ser Tyr Ile Leu 85


316 ACA CAG GGT CCA CTT CCT AAC ACA TGC TGC CAT TTC TGG CTT ATG
    Thr Gln Gly Pro Leu Pro Asn Thr Cys Cys His Phe Trp Leu Met 100


361 GTT TGG CAG CAG AAG ACC AAA GCA GTT GTC ATG CTG AAC CGC ATT
    Val Trp Gln Gln Lys Thr Lys Ala Val Val Met Leu Asn Arg Ile 115
                                    •
406 GTG GAG AAA GAA TCG GTT AAA TGT GCA CAG TAC TGG CCA ACA GAT
    Val Glu Lys Glu Ser Val Lys Cys Ala Gln Tyr Trp Pro Thr Asp 130
                                            •
451 GAC CAA GAG ATG CTG TTT AAA GAA ACA GGA TTC AGT GTG AAG CTC
    Asp Gln Glu Met Leu Phe Lys Glu Thr Gly Phe Ser Val Lys Leu 145


496 TTG TCA GAA GAT GTG AAG TCG TAT TAT ACA GTA CAT CTA CTA CAA
    Leu Ser Glu Asp Val Lys Ser Tyr Tyr Thr Val His Leu Leu Gln 160


541 TTA GAA AAT ATC AAT AGT GGT GAA ACC AGA ACA ATA TCT CAC TTT
    Leu Glu Asn Ile Asn Ser Gly Glu Thr Arg Thr Ile Ser His Phe 175


586 CAT TAT ACT ACC TGG CCA GAT TTT GGA GTC CCT GAA TCA CCA GCT
    His Tyr Thr Thr Trp Pro Asp Phe Gly Val Pro Glu Ser Pro Ala 190


631 TCA TTT CTC AAT TTC TTG TTT AAA GTG AGA GAA TCT GGC TCC TTG
    Ser Phe Leu Asn Phe Leu Phe Lys Val Arg Glu Ser Gly Ser Leu 205
                        •
676 AAC CCT GAC CAT GGG CCT GCG GTG ATC CAC TGT AGT GCA GGC ATT
    Asn Pro Asp His Gly Pro Ala Val Ile His Cys Ser Ala Gly Ile 220
                            •
721 GGG CGC TCT GGC ACC TTC TCT CTG GTA GAC ACT TGT CTT GTT TTG
    Gly Arg Ser Gly Thr Phe Ser Leu Val Asp Thr Cys Leu Val Leu 235


766 ATG GAA AAA GGA GAT GAT ATT AAC ATA AAA CAA GTG TTA CTG AAC
    Met Glu Lys Gly Asp Asp Ile Asn Ile Lys Gln Val Leu Leu Asn 250


811 ATG AGA AAA TAC CGA ATG GGT CTT ATT CAG ACC CCA GAT CAA CTG
    Met Arg Lys Tyr Arg Met Gly Leu Ile Gln Thr Pro Asp Gln Leu 265


856 AGA TTC TCA TAC ATG GCT ATA ATA GAA GGA GCA AAA TGT ATA AAG
    Arg Phe Ser Tyr Met Ala Ile Ile Glu Gly Ala Lys Cys Ile Lys 280
                                    ↑
```

# FIG. 1 CONT.

```
901 GGA GAT TCT AGT ATA CAG AAA CGA TGG AAA GAA CTT TCT AAG GAA
    Gly Asp Ser Ser Ile Gln Lys Arg Trp Lys Glu Leu Ser Lys Glu 295

946 GAC TTA TCT CCT GCC TTT GAT CAT TCA CCA AAC AAA ATA ATG ACT
    Asp Leu Ser Pro Ala Phe Asp His Ser Pro Asn Lys Ile Met Thr 310

991 GAA AAA TAC AAT GGG AAC AGA ATA GGT CTA GAA GAA GAA AAA CTG
    Glu Lys Tyr Asn Gly Asn Arg Ile Gly Leu Glu Glu Glu Lys Leu 325

1036 ACA GGT GAC CGA TGT ACA GGA CTT TCC TCT AAA ATG CAA GAT ACA
     Thr Gly Asp Arg Cys Thr Gly Leu Ser Ser Lys Met Gln Asp Thr 340

1081 ATG GAG GAG AAC AGT GAG AGT GCT CTA CGG AAA CGT ATT CGA GAG
     Met Glu Glu Asn Ser Glu Ser Ala Leu Arg Lys Arg Ile Arg Glu 355

1126 GAC AGA AAG GCC ACC ACA GCT CAG AAG GTG CAG CAG ATG AAA CAG
     Asp Arg Lys Ala Thr Thr Ala Gln Lys Val Gln Gln Met Lys Gln 370

1171 AGG CTA AAT GAG AAT GAA CGA AAA AGA AAA AGG TGG TTA TAT TGG
     Arg Leu Asn Glu Asn Glu Arg Lys Arg Lys Arg Trp Leu Tyr Trp 385

1216 CAA CCT ATT CTC ACT AAG ATG GGG TTT ATG TCA GTC ATT TTG GTT
     Gln Pro Ile Leu Thr Lys Met Gly Phe Met Ser Val Ile Leu Val 400

1261 GGC GCT TTT GTT GGC TGG ACA CTG TTT TTT CAG CAA AAT GCC CTA
     Gly Ala Phe Val Gly Trp Thr Leu Phe Phe Gln Gln Asn Ala Leu 415

1306 TAA ACA ATT AAT TTT GCC CAG CAA GCT TCT GCA CTA GTA ACT GAC
     END

1351 AGT GCT ACA TTA ATC ATA GGG GTT TGT CTG CAG CAA ACG CCT CAT
1396 ATC CCA AAA ACG GTG CAG TAG AAT AGA CAT CAA CCA GAT AAG TGA
1441 TAT TTA CAG TCA CAA GCC CAA CAT CTC AGG ACT CTT GAC TGC AGG
1486 TTC CTC TGA ACC CCA AAC TGT AAA TGG CTG TCT AAA ATA AAG ACA
1531 TTC ATG TTT GTT AAA AAC TGG TAA ATT TTG CAA CTG TAT TCA TAC
1576 ATG TCA AAC ACA GTA TTT CAC CTG ACC AAC ATT GAG ATA TCC TTT
1621 ATC ACA GGA TTT GTT TTT GGA GGC TAT CTG GAT TTT AAC CTG CAC
1666 TTG ATA TAA GCA ATA AAT ATT GTG GTT TTA TCT ACG TTA TTG GAA
1711 AGA AAA TGA CAT TTA AAT AAT GTG TGT AAT GTA TAA TGT ACT ATT
1756 GAC ATG GGC ATC AAC ACT TTT ATT CTT AAG CAT TTC AGG GTA AAT
1801 ATA TTT TAT AAG TAT CTA TTT AAT CTT TTG TAG TTA ACT GTA CTT
1846 TTT AAG AGC TCA ATT TGA AAA ATC TGT TAC TAA AAA AAA AAA TTG
1891 TAT GTC GAT TGA ATT GTA CTG GAT ACA TTT TCC ATT TTT CTA AAG
1936 AGA AGT TTG ATA TGA GCA GTT AGA AGT TGG AAT AAG CAA TTT CTA
1981 CTA TAT ATT GCA TTT CTT TTA TGT TTT ACA GTT TTC CCC ATT TTA
2026 AAA AGA AAA GCA AAC AAA GAA ACA AAA GTT TTT CCT AAA AAT ATC
2071 TTT GAA GGA AAA TTC TCC TTA CTG GGA TAG TCA GGT AAA CAG TTG
2116 GTC AAG ACT TTG TAA AGA AAT TGG TTT CTG TAA ATC CCA TTA TTG
2161 ATA TGT TTA TTT TTC ATG AAA ATT TCA ATG TAG TTG GGG TAG ATT
2206 ATG ATT TAG GAA GCA AAA GTA AGA AGC AGC ATT TTA TGA TTC ATA
2251 ATT TCA GTT TAC TAG ACT GAA GTT TTG AAG TAA ACC C
```

E    X  H   S   E

0.2 KBP

FIG. 2

# FIGURE 3